# FASCICULE DE BREVET EUROPEEN

(11) **EP 0 850 310 B1**
(45) Date de publication et mention de la délivrance du brevet: **15.12.2004**
(21) Numéro de dépôt: 96931124.0
(22) Date de dépôt: 13.09.1996
(51) Int. Cl.: C12N 15/69, C12N 15/70, C12N 1/21, C07K 14/82, A61K 31/70, A61K 48/00

(54) **MOLECULE D'ADN CIRCULAIRE A ORIGINE DE REPLICATION CONDITIONNELLE, LEUR PROCEDE DE PREPARATION ET LEUR UTILISATION EN THERAPIE GENIQUE**
ZIRKULÄRES DNA MOLEKÜL MIT EINEM ABHÄNGIGEN REPLIKATIONSURSPRUNG, SEINE HERSTELLUNGSVERFAHREN UND SEINE VERWENDUNG IN DER GENTHERAPIE
CIRCULAR DNA MOLECULE WITH CONDITIONAL ORIGIN OF REPLICATION, METHOD FOR PREPARING SAME AND USE THEREOF IN GENE THERAPY

(30) Priorité: 15.09.1995 FR 9510825
(43) Date de publication de la demande: 01.07.1998
(60) Demande divisionnaire: 04016978.1
(73) Titulaire: GENCELL S.A., 94400 Vitry sur Seine (FR)
(72) Inventeur: CROUZET, Joel, F-92330 Sceaux (FR); SOUBRIER, Fabienne, F-94320 Thiais (FR)
(74) Mandataire: Warcoin, Jacques
(86) Numéro de dépôt international: PCT/FR1996/001414
(87) Numéro de publication internationale: WO 1997/010343

(56) Documents cités:
- EP-A- 0 416 505
- EP-A- 0 485 701
- WO-A-95/03418
- WO-A-95/30762
- WO-A-96/01899
- US-A- 4 342 832
- US-A- 4 761 367
- US-A- 5 434 065
- GENE TRANSFER AND GENE THERAPY, 1989, ALAN R. LISS, INC., NEW YORK ,US, pages 47-56, XP002004631 N. DALYOT AND A. OPPENHEIM: "Efficient transfer of the complete human beta-globin gene into human and mouse hemopoeitic cells via SV40 pseudovirions"
- GENE, vol. 89, 1990, ELSEVIER SCIENCE PUBLISHERS,B.V.,AMSTERDAM,NL;, pages 211-221, XP002004632 S. RUSCONI ET AL.: "A novel expression assay to study transcriptional activators"
- PROC. NATL.ACAD SCI., vol. 88, December 1991, NATL. ACAD SCI.,WASHINGTON,DC,US;, pages 10686-10690, XP002004633 H.A. VASAVADA ET AL.: "A contingent replication assay for the detection of protein-protein interactions in animal cells"
- CELL, vol. 23, January 1981, CELL PRESS,CAMBRIDGE,MA,US;, pages 175-182, XP000572239 Y GLUZMAN: "SV40-transformed simian cells support the replication of early SV40 mutants"
- J. BACTERIOL., vol. 172, no. 11, November 1990, AM. SOC. MICROBIOL.,BALTIMORE,US;, pages 6557-6567, XP000572232 M. HERRERO ET AL.: "Transposon vectors containing non-antibiotic resistance selection markers for cloning and stable chromosomal insertion of foreign genes in gram-negative bacteria" cited in the application
- GENE, vol. 138, 1994, ELSEVIER SCIENCE PUBLISHERS,B.V.,AMSTERDAM,NL;, pages 1-7, XP002004634 W.W. METCALF ET AL.: "Use of the rep technique for allele replacement to construct new Escherichia coli hosts for maintance of R6Kgamma origin plasmids at different copy numbers" cited in the application
- CELL, vol. 36, April 1984, CELL PRESS,CAMBRIDGE,MA,US;, pages 1097-1103, XP000572222 D.K. SUMMERS AND D.J. SHERRATT: "Multimerization of high copy number plasmids causes instability: ColE1 encodes a determinant essential for plasmid monomerization and stability" cited in the application
- FEBS LETTERS, vol. 228, no. 1, February 1988, ELSEVIER, AMSTERDAM, NL, pages 7-11, XP002004635 M. INUZUKA AND Y. WADA: "An initator protein for plasmid R6K DNA replication"
- GENE, vol. 28, 1994, ELSEVIER SCIENCE PUBLISHERS,B.V.,AMSTERDAM,NL;, pages 45-54, XP002004636 J.E.L. LARSEN ET AL.: "Low-copy-number plasmid-cloning vectors amplifiable by derepression of an inserted foreign promoter"

### LISTE DE SEQUENCES

(1) INFORMATIONS GENERALES:
   (i) DEPOSANT:
      (A) NOM: RHONE POULENC RORER S.A.
      (B) RUE: 20, Avenue Raymond Aron
      (C) VILLE: ANTONY
      (E) PAYS: FRANCE
      (F) CODE POSTAL: 92165
      (G) TELEPHONE: 40.91.69.22
      (H) TELECOPIE: (1) 40.91.72.96

   (ii) TITRE DE L' INVENTION: Molécule d'ADN circulaire à origine de réplication conditionnelle, leur procédé de préparation et leur utilisation en thérapie génique
   (iii) NOMBRE DE SEQUENCES: 6
   (iv) FORME DECHIFFRABLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 389 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 1:
   TGTCAGCCGT TAAGTGTTCC TGTGTCACTG AAAATTGCTT TGAGAGGCTC TAAGGGCTTC 60
   TCAGTGCGTT ACATCCCTGG CTTGTTGTCC ACAACCGTTA AACCTTAAAA GCTTTAAAAG 120
   CCTTATATAT TCTTTTTTTT CTTATAAAAC TTAAAACCTT AGAGGCTATT TAAGTTGCTG 180
   ATTTATATTA ATTTTATTGT TCAAACATGA GAGCTTAGTA CGTGAAACAT GAGAGCTTAG 240
   TACGTTAGCC ATGAGAGCTT AGTACGTTAG CCATGAGGGT TTAGTTCGTT AAACATGAGA 300
   GCTTAGTACG TTAAACATGA GAGCTTAGTA CGTGAAACAT GAGAGCTTAG TACGTACTAT 360
   CAACAGGTTG AACTGCTGAT CTTCAGATC 389
(2) INFORMATIONS POUR LA SEQ ID NO: 2:
   (i) CARACTERISTIQUES DE LA SEQUENCE:
      (A) LONGUEUR: 960 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 2:
   TATACAGAAT GATGAGGTTT TTTTATGAGA CTCAAGGTCA TGATGGACGT GAACAAAAAA 60
   ACGAAAATTC GCCACCGAAA CGAGCTAAAT CACACCCTGG CTCAACTTCC TTTGCCCGCA 120
   AAGCGAGTGA TGTATATGGC GCTTGCTCCC ATTGATAGCA AAGAACCTCT TGAACGAGGG 180
   CGAGTTTTCA AAATTAGGGC TGAAGACCTT GCAGCGCTCG CCAAAATCAC CCCATCGCTT 240
   GCTTATCGAC AATTAAAAGA GGGTGGTAAA TTACTTGGTG CCAGCAAAAT TTCGCTAAGA 300
   GGGGATGATA TCATTGCTTT AGCTAAAGAG CTTAACCTGC CCTTTACTGC TAAAAACTCC 360
   CCTGAAGAGT TAGATCTTAA CATTATTGAG TGGATAGCTT ATTCAAATGA TGAAGGATAC 420
   TTGTCTTTAA AATTCACCAG AACCATAGAA CCATATATCT CTAGCCTTAT TGGGAAAAAA 480
   AATAAATTCA CAACGCAATT GTTAACGGCA AGCTTACGCT TAAGTAGCCA GTATTCATCT 540
   TCTCTTTATC AACTTATCAG GAAGCATTAC TCTAATTTTA AGAAGAAAAA TTATTTTATT 600
   ATTTCCGTTG ATGAGTTAAA GGAAGAGTTA ACAGCTTATA CTTTTGATAA AGATGGAAAT 660
   ATTGAGTACA AATACCCTGA CTTTCCTATT TTTAAAAGGG ATGTGTTAAA TAAAGCCATT 720
   GCTGAAATTA AAAAGAAAAC AGAAATATCG TTTGTTGGCT TCACTGTTCA TGAAAAAGAA 780
   GGAAGAAAAA TTAGTAAGCT GAAGTTCGAA TTTGTCGTTG ATGAAGATGA ATTTTCTGGC 840
   GATAAAGATG ATGAAGCTTT TTTTATGAAT TTATCTGAAG CTGATGCAGC TTTTCTCAAG 900
   GTATTTAATG AAACCGTACC TCCCAAAAAA GCTAAGGGGT GATATATGGC TAAAATTTAC 960
(2) INFORMATIONS POUR LA SEQ ID NO: 3:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 3:
   GACCAGTATT ATTATCTTAA TGAG 24
(2) INFORMATIONS POUR LA SEQ ID NO: 4:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 4:
   GTATTTAATG AAACCGTACC TCCC 24
(2) INFORMATIONS POUR LA SEQ ID NO: 5:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 5:
   CTCTTTTAAT TGTCGATAAG CAAG 24
(2) INFORMATIONS POUR LA SEQ ID NO: 6:
   (i) CARACTERISTIQUES DE LA SEQUENCE:
      (A) LONGUEUR: 24 paires de bases
      (B) TYPE: nucléotide
      (C) NOMBRE DE BRINS: simple
      (D) CONFIGURATION: linéaire
   (ii) TYPE DE MOLECULE: ADNc
   (xi) DESCRIPTION DE LA SEQUENCE: SEQ ID NO: 6:
   GCGACGTCAC CGAGGCTGTA GCCG 24

## Description

La présente invention concerne une nouvelle molécule d'ADN à réplication conditionnelle, utilisable en thérapie génique ou pour la production de protéines recombinantes.

La thérapie génique consiste à corriger une déficience ou une anomalie en introduisant une information génétique dans la cellule ou l'organe affecté. Cette information peut être introduite soit in vitro dans une cellule extraite de l'organe et ensuite réinjectée dans l'organisme, soit in vivo, directement dans le tissu visé. S'agissant d'une molécule de haut poids moléculaire et de charge négative, l'ADN a des difficultés pour traverser spontanément les membranes cellulaires phospholipidiques. Différents vecteurs sont donc utilisés afin de permettre le transfert de gène : les vecteurs viraux d'une part, les vecteurs chimiques et/ou biochimiques, naturels ou synthétiques, d'autre part.

Les vecteurs viraux (rétrovirus, adénovirus, virus adéno-associés, ...) sont très efficaces, notamment pour le passage des membranes, mais présentent un certain nombre de risques tels que la pathogénicité, la recombinaison, la réplication, l'immunogénicité, ...

Les vecteurs chimiques et/ou biochimiques permettent d'éviter ces risques (pour revues, voir Behr, 1993, Cotten et Wagner, 1993). Ce sont par exemple des cations (phosphate de calcium, DEAE-dextran ...) qui agissent en formant des précipités avec l'ADN, lesquels peuvent être "phagocytés" par les cellules. Il peut également s'agir de liposomes dans lesquels l'ADN est incorporé et qui fusionnent avec la membrane plasmique. Les vecteurs synthétiques de transfert de gènes sont généralement des lipides ou des polymères cationiques qui complexent l'ADN et forment avec lui une particule portant des charges positives en surface. A titre illustratif de cetype de vecteurs, on peut notamment citer le dioctadécylamidoglycylspermine (DOGS, Transfectam™) ou le chlorure de N-[1-(2,3-dioleyloxy)propyl]-N,N,N-triméthylammonium (DOTMA, Lipofectin™).

Toutefois, l'utilisation de vecteurs chimiques et/ou biochimiques ou d'ADN nu implique la possibilité de produire des quantités importantes d'ADN de pureté pharmacologique. En effet, dans les techniques de thérapie génique, le médicament est constitué par l'ADN même et il est essentiel de pouvoir fabriquer, dans des quantités adaptées, des ADN ayant des propriétés appropriées à un usage thérapeutique chez l'homme.

Dans le cas de la vectorologie non virale, ce sont des plasmides d'origine bactérienne qui sont mis en oeuvre. Les plasmides généralement utilisés en thérapie génique portent (i) une origine de réplication, (ii) un gène marqueur tel qu'un gène de résistance à un antibiotique (kanamycine, ampicilline...) et (iii) un ou plusieurs transgènes avec des séquences nécessaires à leur expression (enhanceur(s), promoteur(s), séquences de polyadénylation...).

Toutefois, la technologie actuellement disponible ne donne pas entière satisfaction.

D'une part, il demeure un risque de dissémination dans l'organisme. C'est ainsi qu'une bactérie présente dans l'organisme peut, à une fréquence faible, recevoir ce plasmide. Ceci a d'autant plus de chances de se passer qu'il s'agit d'un traitement de thérapie génique in vivo dans lequel l'ADN peut être disséminé dans l'organisme du patient et peut se trouver au contact de bactéries qui infectent ce patient ou bien de bactéries de la flore commensale. Si la bactérie receveuse du plasmide est une entérobactérie, telle qu'E. coli, ce plasmide peut se répliquer. Un tel événement conduit alors à la dissémination du gène thérapeutique. Dans la mesure où les gènes thérapeutiques utilisés dans des traitements de thérapie génique peuvent coder par exemple pour une lymphokine, un facteur de croissance, un antioncogène, ou une protéine dont la fonction fait défaut chez l'hôte et permet donc de corriger un défaut génétique, la dissémination de certains de ces gènes pourrait avoir des effets imprévisibles et préoccupants (par exemple si une bactérie pathogène acquérait le gène d'un facteur de croissance humain).

D'autre part, les plasmides utilisés généralement en thérapie génique non virale possèdent aussi un marqueur de résistance à un antibiotique (ampicilline. kanamycine...). La bactérie acquérant un tel plasmide a donc un avantage sélectif indéniable puisque tout traitement antibiothérapique, utilisant un antibiotique de la même famille que celui sélectionnant le gène de résistance du plasmide, va conduire à la sélection du plasmide en question. A cet égard, l'ampicilline fait partie des β-lactames qui est la famille d'antibiotiques les plus utilisés au monde. L'utilisation de marqueurs de sélection chez la bactéries qui ne soient pas des gènes de résistance à des antibiotique serait donc particulièrement avantageuse. Elle éviterait la sélection de bactéries ayant pu recevoir un plasmide portant un tel marqueur.

Il est donc particulièrement important de chercher à limiter au maximum la dissémination des gènes thérapeutiques et des gènes de résistance.

La présente invention a précisément pour objet de proposer de de nouvelles molécules d'ADN, utilisables en thérapie génique ou pour la production de protéines recombinantes in vitro, ne se répliquant que dans des cellules pouvant complémenter certaines fonctions de ces vecteurs non viraux.

L'invention concerne également une méthode particulièrement efficace pour la préparation de ces molécules d'ADN.

Les molécules d'ADN revendiquées ont pour avantage d'éliminer les risques liés à une dissémination du plasmide, tels que (1) la réplication et la dissémination, pouvant entraîner une surexpression non contrôlée du gène thérapeutique, (2) la dissémination et l'expression de gènes de résistance. L'information génétique contenue dans les molécules d'ADN selon l'invention comprend en effet le(s) gène(s) thérapeutique(s) et les signaux de régulation de son (leur) expression, une origine de réplication conditionnelle fonctionnelle limitant très fortement le spectre d'hôte cellulaire de ce plasmide, un marqueur de sélection de taille réduite de préférence différent d'un gène conférant la résistance à un antibiotique et le cas échéant, un fragment d'ADN permettant la résolution de multimères de plasmide. La probabilité que ces molécules (et donc l'information génétique qu'elles contiennent) soient transférées à un microorganisme, et maintenues de manière stable, est très limitée.

Enfin les vecteurs selon l'invention, également désignées miniplasmides en raison de leur structure circulaire, de leur taille réduite et de leur forme superenroulée, présentent les avantages supplémentaires suivants: En raison de leur taille réduite par rapport aux plasmides dérivés de ColE1 classiquement utilisés, les molécules d'ADN selon l'invention ont potentiellement une meilleure biodisposition in vivo . En particulier, elles présentent des capacités de pénétration et de distribution cellulaires améliorées. Ainsi, il est reconnu que le coefficient de diffusion dans les tissus est inversement proportionnel au poids moléculaire (Jain, 1987). De même, au niveau cellulaire, les molécules de haut poids moléculaire ont une moins bonne perméabilité à travers la membrane plasmique. En outre, pour le passage du plasmide au noyau, indispensable à son expression, le poids moléculaire élevé est également un inconvénient, les pores nucléaires imposant une limite de taille pour la diffusion vers le noyau (Landford et al., 1986). La réduction de taille des parties non-thérapeutiques de la molécule d'ADN (origine de réplication et gène de sélection notamment) selon l'invention permet également de diminuer la taille des molécules d'ADN. La partie permettant la réplication et la sélection de ce plasmide chez la bactérie (1,1 kb) est diminuée par un facteur 3, en comptant par exemple 3 kb pour l'origine de réplication et le marqueur de résistance partie vecteur. Cette diminution (i) de poids moléculaire et (ii) de charge négative, confère aux molécules de l'invention des capacités améliorées de diffusion et de biodisponibilité tissulaires, cellulaires et nucléaires.

Plus précisément, la présente invention concerne une moléculaire d'ADN sous forme circulaire, utile en thérapie génique, comprenant au moins une séquence nucléique d'intérêt, et également les signaux de régulation de son expression dont la transcription et éventuellement la traduction dans la cellule cible eucaryote génèrent des produits ayant un intérêt thérapeutique, vaccinal, agronomique ou vétérinaire comprenant :
a. la région permettant sa réplication comprend une origine de réplication conditionnelle issue d'un plasmide ou d'un bactériophage dont la fonctionnalité dans une cellule procaryote hôte requiert la présence d'au moins une protéine initiatrice de réplication spécifique dudit plasmide ou bactériophage et étrangère à ladite cellule procaryote hôte,
b. ladite molécule d'ADN ne comporte pas ladite protéine initiatrice de réplication,
c. ladite molécule d'ADN comporte un marqueur de sélection de taille réduite différent d'un gène conférant la résistance à un antibiotique, et
d. ladite molécule d'ADN ne se réplique que dans les cellules pouvant complémenter ladite protéine initiatrice.

Cette molécule d'ADN peut se présenter sous forme mono ou double brin et avantageusement possède une forme superenroulée.

Au sens de la présente invention, les cellules hôtes mises en oeuvre peuvent être de diverses origines. Il peut s'agir de cellules eucaryotes ou procaryotes. Selon un mode de réalisation privilégiée de l'invention, il s'agit de cellules procaryotes.

Classiquement, la réplication des plasmides bactéries nécessite la présence d'au moins une protéine codée par l'hôte cellulaire de type ARN polymérase. Rnase, ADN polymérase... Pour les raisons déjà exposées précédemment, on ne peut totalement s'affranchir, avec ce type de réplication, d'éventuels risques de dissémination dans l'organisme traité. Avantageusement, la fonctionnalité de l'origine de réplication de la molécule d'ADN selon l'invention exige la présence d'une protéine spécifique et étrangère à la cellule hôte. Cette caractéristique a pour intérêt de réduire le spectre d'hôte du plasmide revendiqué à des souches spécifiques exprimant cette protéine initiatrice. La molécule d'ADN, mise au point dans le cadre de la présente invention, possède donc avantageusement une origine de réplication dite conditionnelle.

L'origine de réplication conditionnelle mise en oeuvre selon la présente invention peut provenir de plasmides ou bactériophages, partageant les caractéristiques suivantes: ils contiennent dans leur origine de réplication des séquences répétées, ou itérons et ils codent pour au moins une protéine initiatrice de la réplication (Rep) qui leur est spécifique. A titre d'exemple, on peut citer les systèmes de réplication conditionnelle des plasmides et bactériophages suivants :

| **plasmide ou bactériophage** | **protéine initiatrice spécifique** |
|---|---|
| RK2 (Stalker et al., 1981) | TrfA |
| R1 (Ryder et al., 1981) | RepA |
| pSC101 (Vocke and Bastia, 1983) | RepA |
| F (Murotsu et al., 1981) | protéine E |
| Rts1 (Itoh et al., 1982,1987) | RepA |
| RSF1010 (Miao et al., 1995) | RepC |
| P1 (Abeles et al., 1984) | RepA |
| P4 (Flensburg and Calendar, 1987) | protéine alpha |
| lambda (Moore et al., 1981) | protéine O |
| phi 82 (Moore et al., 1981) | protéine O de phi 82 |
| phi 80 | protéine O de phi 80 |

Selon un mode de réalisation préféré de l'invention, l'origine de réplication mise en oeuvre dans les molécules d'ADN revendiquées est issue d'un plasmide naturel d'E. coli appelé R6K.

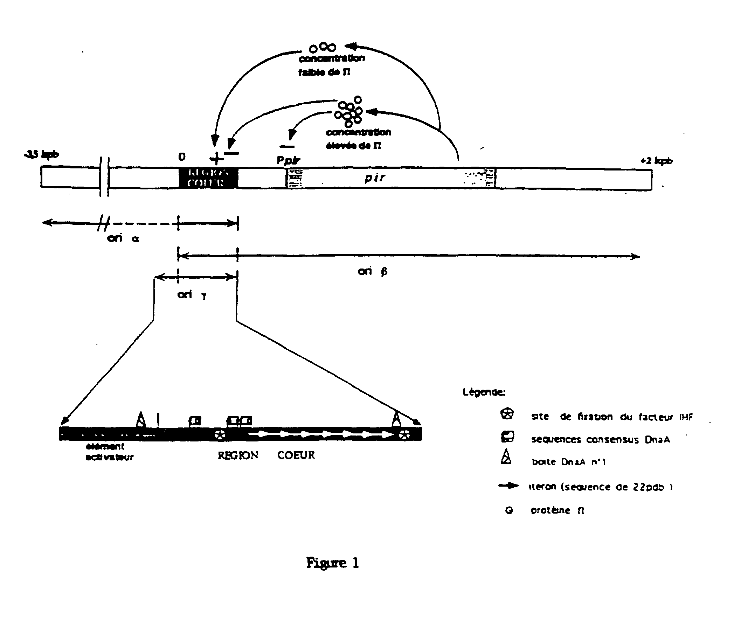

Les fonctions de réplication de R6K sont regroupées dans un fragment d'ADN de 5,5 kpb (figure 1) comprenant 3 origines de réplication α, β et γ (γ et β assurant 90 % de la réplication) et un opéron codant pour les protéines initiatrices de réplication π et la protéine Bis. L'information génétique minimale nécessaire au maintien de ce plasmide à son nombre de copies caractéristique (15 copies par génome) est contenue dans deux éléments : les 400 pdb de l' ori γ et le gène *pir,* dont le produit est la protéine initiatrice π.

L'ori γ peut être divisée en deux parties fonctionnelles : la partie-coeur et l'élément activateur (figure 1). La partie-coeur, essentielle pour la réplication contient les itérons (7 répétitions directes de 22 pdb) où se lie la protéine π représenté en SEQ ID N°1, et des segments flanquants, cibles de protéines de l'hôte (IHF, DnaA).

Selon un mode préféré de l'invention, l'origine de réplication du vecteur revendiqué est constituée en tout ou partie par cette origine de réplication γ du plasmide R6k et plus préférentiellement en tout ou partie par la SEQ ID N°1 ou l'un de ses dérivés.

Au sens de la présente invention, le terme dérivé désigne toute séquence différant de la séquence considérée en raison d'une dégénérescence du code génétique, obtenue par une ou plusieurs modifications de nature génétique et/ou chimique, ainsi que toute séquence hybridant avec ces séquences ou des fragments de celles-ci et dont le produit possède l'activité indiquée à l'égard de la protéine initiatrice de la réplication, π. Par modification de nature génétique et/ou chimique, on peut entendre toute mutation, substitution, délétion, addition et/ou modification d'un ou plusieurs résidus. Le terme dérivé comprend également les séquences homologues à la séquence considérée, issues d'autres sources cellulaires et notamment de cellules d'origine humaine, ou d'autres organismes, et possédant une activité de même type. De telles séquences homologues peuvent être obtenues par des expériences d'hybridation. Les hybridations peuvent être réalisées à partir de banques d'acides nucléiques, en utilisant comme sonde la séquence native ou un fragment de celle-ci, dans des conditions de stringence conventionnelles (Maniatis et al., Cf techniques générales de biologie moléculaire), ou, de préférence, dans des conditions de stringence élevées.

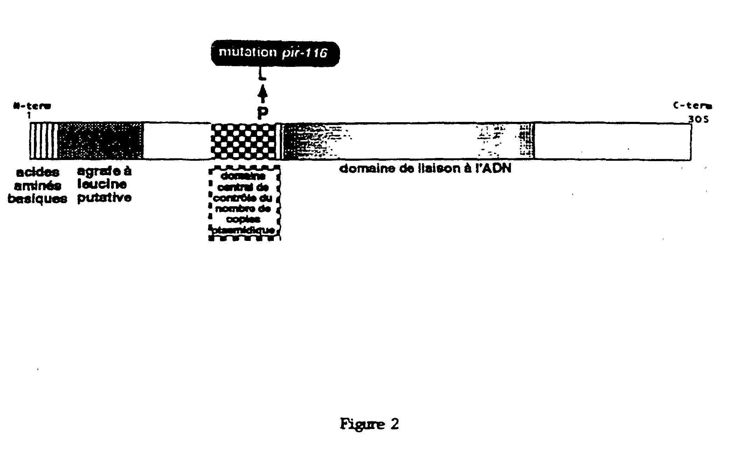

L'origine de réplication décrite ci-dessus qui présente l'avantage d'être de taille très limitée, est fonctionnelle uniquement en présence d'une protéine initiatrice spécifique, la protéine Pi, produit du gène pir ( SEQ ID N°2). Cette protéine pouvant agir en trans, il est possible de dissocier physiquement l'ori gamma du gène pir, qui pourra être introduit dans le génome de la cellule choisie comme hôte spécifique de ces plasmides. Des mutations dans π peuvent altérer ses fonctions inhibitrices (Inuzuka et Wada, 1985 ) et entraîner une augmentation du nombre de copies des dérivés de R6K, jusqu'à plus de 10 fois le nombre de copies initial. Ces substitutions sont toutes comprises dans un domaine de 40 acides aminés, qui semble donc responsable du contrôle par π du nombre de copies plasmidiques (figure 2).

Selon un mode de réalisation avantageux de la présente invention, la protéine π, exprimée dans la cellule hôte, résulte de l'expression du gène représenté en SEQ ID N°2 ou l'un de ses dérivés tels que définis précédemment et plus particulièrement du gène pir 116 qui comprend une mutation par rapport au gène pir. Cette mutation correspond à la substitution d'une proline par une leucine. Dans ce contexte, le nombre de copies des dérivés de R6K est de l'ordre de 250 copies par génome.

Outre une origine de réplication conditionnelle telle que définie précédemment, les molécules d'ADN revendiquées contiennent une région comprenant un (ou plusieurs) gène(s) permettant d'assurer la sélection de la molécule d'ADN chez l'hôte choisi.

Il peut s'agir d'un marqueur classique de type gène conférant une résistance à un antibiotique, tels la kanamycine, l'ampiciline, le chloramphénicol, la streptomycine, la spectinomycine, la lividomycine ou autres.

Toutefois, selon un mode de réalisation privilégié de l'invention, cette région est différente d'un gène conférant une résistance à un antibiotique. Il peut ainsi s'agir d'un gène dont le produit est indispensable à la viabilité de l'hôte envisagé, dans des conditions de cultures définies. Il peut-être par exemple :
- un gène codant pour un ARNt suppresseur, d'origine naturelle ou synthétique. Il s'agit plus préférentiellement d'un ARNt de codon ambre (TAG)
- un gène dont le produit est nécessaire au métabolisme de la cellule, dans certaines conditions de culture: gène intervenant dans la biosynthèse d'un métabolite (acide aminé, vitamine...), gène de catabolisme permettant d'assimiler une substance présente dans le milieu de culture (source d'azote ou de carbone particulières)...

Selon un mode privilégié de l'invention, cette région contient une cassette d'expression d'un gène codant pour un ARNt suppresseur de codons spécifiques. Celui-ci peut être notamment choisi parmi ceux codant pour les bases Phénylalanine, Cystéine, Proline, Alanine et Histidine. Il s'agit plus préférentiellement d'un ARNt suppresseur des codons ambre (TAG).

Dans ce cas particulier, le système utilisé pour sélectionner, chez les hôtes cellulaires, les molécules d'ADN sujets de la présente invention comporte deux éléments: 1)sur la molécule d'ADN, un gène codant pour un ARN de transfert suppresseur de codon ambre (TAG) qui constitue le marqueur de sélection, dit gène (sup) et 2) un hôte spécifique dont un des gènes, essentiel dans certaines conditions de culture, contient un codon ambre TAG. Cette cellule pourra croître, dans les conditions de culture pour lesquelles le produit du gène contenant le codon TAG est essentiel, uniquement si le plasmide permettant l'expression de sup est présent dans la cellule. Les conditions de culture constituent donc la pression de sélection de la molécule d'ADN. Les gènes sup utilisés peuvent être d'origine naturelle (Glass et al., 1982) ou provenir de construction synthétique (Normanly et al., 1986; Kleina et al., 1990).

Un tel système offre une grande flexibilité dans la mesure où, suivant le gène comportant une mutation ambre, il est possible de déterminer différents milieux sélectifs. Chez la bactérie *Lactococcus lactis,* par exemple, le codon ambre est localisé dans un gène de biosynthèse des purines. Ceci permet la sélection du plasmide porteur du gène codant pour l'ARNt suppresseur lorsque les bactéries se multiplient dans le lait. Un tel marqueur a l'avantage d'être de taille très réduite et de ne pas contenir de séquences "étrangères", provenant de phages ou de transposons.

Selon un mode de réalisation particulier de l'invention, la molécule d'ADN comprend en outre un fragment d'ADN, cible de recombinases site-spécifiques, qui permet la résolution des multimères de plasmides.

Ainsi, un tel fragment, introduit sur une molécule d'ADN circulaire et dont l'origine de réplication est par exemple ori gamma permet de résoudre les multimères d'un tel plasmide. De tels multimères sont notamment observés lorsque la molécule d'ADN est préparée dans une souche portant un allèle muté de pir qui permet d'augmenter le nombre de copies des dérivés de R6K, comme pir-116.

Cette recombinaison peut être réalisée grâce à divers systèmes qui entraînent la recombinaison site-spécifique entre des séquences. Plus préférentiellement, la recombinaison site-spécifique de l'invention est obtenue au moyen de séquences de recombinaison intramoléculaire spécifique qui sont capables de recombiner entre elles en présence de protéines spécifiques, généralement désignée recombinase. Dans ce cas précis, il s'agit des recombinases XerC et XerD. Pour cette raison, les molécules d'ADN selon l'invention comprennent généralement en outre une séquence permettant cette recombinaison site spécifique. Le système de recombinaison spécifique présent dans les constructions génétiques selon l'invention (recombinases et site de reconnaissance spécifique) peut être de différentes origines. En particulier, les séquences spécifiques et les recombinases utilisées peuvent appartenir à différentes classes structurales, et notamment à la famille de résolvase du transposon Tn3 ou à la famille de l'intégrase du bactériophage lambda. Parmi les recombinases appartenant à la famille du transposon Tn3, on peut citer notamment la résolvase du transposon Tn3 ou des transposons, Tn21 et Tn522 (Stark et al., 1992) ; l'invertase Gin du bactériophage mu ou encore les résolvases de plasmides, telle que celle du fragment par de RP4 (Abert et al., Mol. Microbiol. 12 (1994) 131). Parmi les recombinases appartenant à la famille de l'intégrase du bactériophage X, on peut citer notamment l'intégrase des phages lambda (Landy et al., Science 197 (1977) 1147), P22 et ϕ80 (Leong et al., J. Biol. Chem, 260 (1985) 4468), HP1 de Haemophilus influenzae (Hauser et al., J. Biol. Chem. 267 (1992) 6859), l'intégrase Cre du phage P1, l'intégrase du plasmide pSAM2 (EP 350 341) ou encore la FLP recombinase du plasmide 2µ et les recombinases XerC et XerD d'E coli.

Préférentiellement, les molécules d'ADN objets de la présente invention contiennent le fragment cer du plasmide naturel d'E. coli Co1E1. Le fragment cer utilisé est un fragment HpaII de 382 pdb de ColE1 dont il a été montré qu'il permettait, en cis, la résolution de multimères de plasmides (Summers et al., 1984 ; Leung et al., 1985). Il est aussi possible d'utiliser un fragment HpaII-TaqI de taille plus réduite (280 pdb) ou un fragment plus petit (environ 220 pdb), compris dans le fragment HpaII, et qui possèdent les mêmes propriétés (Summers and Sherratt, 1988). Cette résolution passe par une recombinaison intramoléculaire spécifique, qui fait intervenir quatre protéines codées par le génome d'E. coli: ArgR, PepA, XerC et XerD (Stirling et al., 1988,1989 ; Colloms et al., 1990 ; Blakely et al., 1993)

A ce titre, il particulièrement avantageux d'utiliser tout ou partie du fragment *cer* de ColE1 ou de l'un de ses dérivés tels que définis précédemment.

Selon une variante de mise en oeuvre, les molécules d'ADN de l'invention peuvent comprendre en outre une séquence capable d'interagir spécifiquement avec un ligand. De manière préférentielle, il s'agit d'une séquence capable de former, par hybridation, une triple-hélice avec un oligonucléotide spécifique. Cette séquence permet ainsi de purifier les molécules de l'invention par hybridation sélective avec un oligonucléotide complémentaire immobilisé sur un support (voir la demande WO96/18744). La séquence peut être positionnée en tout site de la molécule d'ADN de l'invention, dès lors qu'elle n'affecte pas la fonctionnalité du gène d'intérêt et de l'origine de réplication.

A titre de molécule d'ADN représentative de la présente invention, on peut tout particulièrement revendiquer le plasmide pXL2774 et ses dérivés. Au sens de l'invention on entend par dérivé toute construction dérivant du pXL2774 et comportant un ou plusieurs gènes d'intérêt autres que le gène luciférase. On peut également citer les plasmides pXL3029 et 3030 comportant une cassette d'expression d'un gène thérapeutique et une séquence capable d'interagir spécifiquement avec un ligand.

La présente invention se rapporte également à la mise au point d'un procédé, de constructions d'hôtes cellulaires spécifiques, particulièrement efficaces pour la production de ces molécules d'ADN thérapeutiques.

Un autre objet de la présente invention se rapporte à un procédé de production de molécule d'ADN circulaire caractérisé en ce que l'on cultive une cellule hôte contenant au moins un molécule d'ADN telle que définie précédemment et une protéine, exprimée in situ ou non, conditionnant la fonctionnalité de l'origine de réplication deladite molécule d'ADN, spécifique et étrangère à ladite cellule hôte, dans des conditions permettant la sélection des cellules hôtes transformées par lesdites molécules d'ADN.

Plus préférentiellement, la protéine conditionnant la fonctionnalité de l'origine de réplication de la molécule d'ADN est exprimée in situ à partir d'un gène correspondant. Le gène codant pour la protéine initiatrice de la réplication peut être porté par un réplicon annexe, compatible avec les dérivés de l'origine de réplication conditionnelle utilisée ou bien introduit dans le génome de la cellule-hôte par recombinaison, grâce à un transposon, un bactériophage ou tout autre vecteur. Dans le cas particulier où le gène exprimant la protéine est placé sur un réplicon annexe, ce dernier contient également une région promotrice de la transcription fonctionnelle dans la cellule, ainsi qu'une région située en 3', et qui spécifie un signal de fin transcriptionnelle. Concernant la région promotrice, il peut s'agir d'une région promotrice naturellement responsable de l'expression du gène considéré lorsque celle-ci est susceptible de fonctionner dans la cellule. Il peut également s'agir de régions d'origine différente (responsables de l'expression d'autres protéines, ou même synthétiques). Notamment, il peut s'agir de séquences promotrices de gènes procaryotes ou bactériophages. Par exemple, il peut s'agir de séquences promotrices issues du génome de la cellule.

A titre de gènes codant pour la protéine initiatrice de la réplication, pourront-être utilisés soit les gènes sauvages, soit des allèles mutés permettant d'obtenir un nombre de copies augmenté des plasmides (ou dérivés) spécifiques de la protéine initiatrice conditionnant la fonctionnalité de l'origine de réplication mise en oeuvre dans la molécule d'ADN.

De tels mutants ont été notamment décrits pour les plasmides R6K (Inuzuka and Wada, 1985 ; Greener et al., 1990), Rts1 (Terawaki and Itoh, 1985 ; Terawaki et al, 1990 ; Zeng et al., 1990), F (Seelke et al., 1982 ; Helsberg et al., 1985 ; Kawasaki et al., 1991), RK2 (Durland et al., 1990 ; Haugan et al., 1992, 1995), pSe101 (Xia et al., 1991 ; Goebel et al., 1991; Fang et al., 1993).

Dans le cas particulier où la molécule d'ADN mise en oeuvre possède une origine de réplication dérivant du plasmide R6k, la protéine initiatrice est ou dérive de la protéine π de ce même plasmide. Il est particulièrement avantageux d'exprimer une forme mutée de cette protéine capable d'augmenter notablement le nombre de copies initiales. Pour ce faire, le gène intégré au niveau de la cellule hôte est de préférence représenté par tout ou partie de la séquence représentée en SEQ ID N°2 ou l'un de ses dérivés et plus préférentiellement par le gène pir116. La mutation associée, correspond à la substitution d'une proline par une leucine. Selon un mode de réalisation particulier de l'invention, ce gène pir116 est directement incorporé dans le génome de la cellule hôte.

Avantageusement, un des gènes de l'hôte cellulaire spécifique, indispensable dans les conditions. de culture choisies contient un codon spécifique, reconnaissable par l'ARNt suppresseur sélectionné au niveau de la molécule d'ADN. Selon un mode privilégié de l'invention, il s'agit d'un codon ambre TAG. Dans ce cas particulier, la cellule pourra croître, dans les conditions de culture pour lesquelles le produit du gène contenant le codon TAG est essentiel, uniquement si le plasmide permettant l'expression de sup est présent dans la cellule hôte. Les conditions de culture constituent donc la pression de sélection de la molécule d'ADN.

De préférence, le gène comportant le codon ambre est un gène intervenant dans la biosynthèse d'un acide aminé, l'arginine. Ce gène, argE, code pour une N-acétylomithinase (Meinnel et al., 1992) et comporte dans ce cas un codon TAG correspondant à une mutation ponctuelle Gln-53 (CAG)-> TAG ; la pression de sélection du plasmide portant le gène sup est alors assurée par culture en milieu minimum M9 (Maniatis et al.,1989). Toutefois ce pourrait être aussi, par exemple, un gène de biosynthèse d'une vitamine, d'une base nucléique ou bien un gène permettant d'utiliser une source de carbone ou d'azote particulière ou tout autre gène dont la fonctionnalité est indispensable à la viabilité cellulaire dans les conditions de culture choisies.

La cellule hôte est de préférence choisie parmi les souches E.coli et représentée plus préférentiellement par la souche E. coli XAC-1.

Selon un mode de réalisation particulier de l'invention, la cellule hôte mise en oeuvre dans le procédé revendiqué est une cellule de la souche E. coli XAC-1, comprenant dans son génome le gène pir 116 et transformée par le plasmide pXL2774 ou l'un de ses dérivés.

Selon une variante avantageuse de l'invention, la cellule hôte mise en oeuvre dans le procédé revendiqué est une cellule procaryote dans laquelle le gène endA1 ou un gène homologue est inactivé. Le gène endA code pour l'endonucléase I d'E. coli. Cette enzyme périplasmique possède une activité de coupure non spécifique de l'ADN double brin (Lehrnan, I. R., G. G. Roussos and E. A. Pratt (1962) J. Biol. Chem. 237:819-828 ; Wright M. (1971) J. Bacteriol. 107:87-94). Une étude menée sur différentes souches de Escherichia coli (sauvage ou endA) a montré que que la dégradation de l'ADN plasmidique incubé dans les extraits de ces souches bactériennes existait dans les souches endA+ mais pas dans les mutants endA (Wnendt S. (1994) BioTechniques 17:270-272). La qualité de l'ADN plasmidique isolé de souches endA+ ou de mutants endA a été étudiée par la société Promega en utilisant leur systéme de purification (Shoenfeld, T., J. Mendez, D. Storts, E. Portman, B.†Patterson, J. Frederiksen and C. Smith. 1995. Effects of bacterial strains carrying the endA1 genotype on DNA quality isolated with Wizard plasmid purification systems. Promega notes 53). Leur conclusion est la suivante : la qualité de l'ADN préparé à partir de mutants endA est globalement meilleure que celle de l'ADN préparé dans les souches endA+ testées.

La qualité des préparations d'ADN plasmidique est donc affectée par toute contamination par cette endonucléase (dégradation de l'ADN à plus ou moins long terme).

La délétion ou la mutation du gène endA peut être envisagée sans problème dans la mesure ou les mutants ne présentant plus cette activité endonucléase se comportent globalement comme les bactéries sauvages (Dürwald, H. and H. Hoffmann-Berling (1968) J. Mol. Biol. 34:331-346).

Le gène endA1 peut être inactivé par mutation, délétion totale ou partielle, disruption, etc. L'inactivation du gène endA de la souche d'E. coli choisie pour produire les plasmides pCOR peut plus particulièrement être réalisée en transférant, grâce au bactériophage P1, la délétion ΔendA::Tc^{R} décrite par Cherepanov et Wackernagel (Cherepanov, P. P. and W. Wackemagel. 1995. Gene disruption in Escherichia coli : Tc^{R} and Km^{R} cassettes with the option of Flp-catalyzed excision of the antibiotic-resistance déterminant. Gene 158:9-1-4) ou en échangeant l'allèle sauvage présent dans le génome de la bactérie d'intérêt avec un allèle muté ou délété de endA et ceci par recombinaison homologue. L'utilisation de ce type de souche dans le cadre de la présente invention permet avantageusement d'améliorer la qualité de l'ADN produit.

L'invention concerne également toute cellule recombinante contenant une molécule d'ADN telle que définie ci-avant. Il peut s'agir de cellule d'origines diverses, de type eucaryotique, procaryotique....

Ces cellules sont obtenues par toute technique connue de l'homme du métier permettant l'introduction dudit plasmide dans une cellule donnée. Il peut s'agir notamment de transformation, électroporation, conjugaison, fusion de protoplastes ou toute autre technique connue de l'homme de l'art.

Les molécules d'ADN selon l'invention peuvent être utilisées dans toute application de vaccination ou de thérapie génique et cellulaire, pour le transfert d'un gène à un organisme, un tissu ou une cellule donnée, ou pour la production de protéines recombinantes in vitro.

En particulier, elles peuvent être utilisées pour une administration directe in vivo, ou pour la modification de cellules in vitro ou ex vivo, en vue de leur implantation à un patient.

A cet égard, un autre objet de la présente invention concerne toute composition pharmaceutique comprenant au moins une molécule d'ADN telle que définie ci-avant. Cette molécule peut y être ou non associée à un vecteur chimique et/ou biochimique de transfection. Il peut s'agir notamment de cations (phosphate de calcium, DEAE-dextran,..), de liposomes. Les vecteurs synthétiques associés peuvent être des lipides ou polymères cationiques. On peut citer comme exemples de tels vecteurs le DOGS (Transfectam TM) ou le DOTMA (lipofectin TM).

Les compositions pharmaceutiques selon l'invention peuvent être formulées en vue d'administrations par voie topique, orale, parentérale, intranasale, intraveineuse, intramusculaire, sous-cutanée, intraoculaire, transderrnique, etc. De préférence, le plasmide revendiqué est utilisé sous une forme injectable ou en application. il peut être mélangé à tout véhicule pharmaceutiquement acceptable pour une formulation injectable, notamment pour une injection directe au niveau du site à traiter. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Il peut s'agir notamment de tampons Tris ou PBS dilués dans du glucose ou du chlorure de sodium. Une injection directe dans la région atteinte du patient est intéressante car elle permet de concentrer l'effet thérapeutique au niveau des tissus affectés. Les doses utilisées peuvent être adaptées en fonction de différents paramètres, et notamment en fonction du gène, du vecteur, du mode d'administration utilisé, de la pathologie concernée ou encore de la durée du traitement recherchée.

Les molécules d'ADN de l'invention peuvent comporter un ou plusieurs gènes d'intérêt, c'est-à-dire un ou plusieurs acides nucléiques (ADNc, ADNg, ADN synthétique ou semi-synthétique, etc) dont la transcription et éventuellement la traduction dans la cellule cible génèrent des produits ayant un intérêt thérapeutique, vaccinal, agronomique ou vétérinaire.

Parmi les gènes d'intérêt thérapeutique, on peut citer plus particulièrement les gènes codant pour des enzymes, les dérivés sanguins, les hormones, les lymphokines : interleukines, interférons, TNF, etc (FR 9203120), les facteurs de croissance, les neurotransmetteurs ou leurs précurseurs ou enzymes de synthèse, les facteurs trophiques : BDNF, CNTF, NGF, IGF, GMF, aFGF, bFGF, NT3, NT5, etc ; les apolipoprotéines : ApoAI, ApoAIV, ApoE, etc (FR 93 05125), la dystrophine ou une minidystrophine (FR 9111947), les gènes suppresseurs de tumeurs : p53, Rb, Rap1A, DCC, k-rev, etc (FR 93 04745), les gènes codant pour des facteurs impliqués dans la coagulation : Facteurs VII, VIII, IX, etc, les gènes suicides : Thymidine kinase, cytosine désarninase, etc; ou encore tout ou partie d'une immunoglobuline naturelle ou artificielle (Fab, ScFv, etc), un ARN ligand (WO91/19813) etc. Le gène thérapeutique peut également être un gène ou une séquence antisens, dont l'expression dans la cellule cible permet de contrôler l'expression de gènes ou la transcription d'ARNm cellulaires. De telles séquences peuvent par exemple être transcrites, dans la cellule cible, en ARN complémentaires d'ARNm cellulaires et bloquer ainsi leur traduction en protéine, selon la technique décrite dans le brevet EP 140 308.

Le gène d'intérêt peut aussi être un gène vaccinant, c'est-à-dire un gène codant pour un peptide antigénique, capable de générer chez l'homme ou l'animal une réponse immunitaire, en vue de la réalisation de vaccins. Il peut s'agir notamment de peptides antigéniques spécifiques du virus d'epstein barr, du virus HIV, du virus de l'hépatite B (EP 185 573), du virus de la pseudo-rage, ou encore spécifiques de tumeurs (EP 259 212).

Généralement, dans les molécules d'ADN de l'invention, le gène d'intérêt thérapeutique, vaccinal, agronomique ou vétérinaire contient également une région promotrice de la transcription fonctionnelle dans la cellule ou l'organisme cible, ainsi qu'une région située en 3', et qui spécifie un signal de fin transcriptionnelle et un site de polyadénylation. Concernant la région promotrice, il peut s'agir d'une région promotrice naturellement responsable de l'expression du gène considéré lorsque celle-ci est susceptible de fonctionner dans la cellule ou l'organisme concernés. D peut également s'agir de régions d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule cible. Parmi les promoteurs eucaryotes, on peut utiliser tout promoteur ou séquence dérivée stimulant ou réprimant la transcription d'un gène de façon spécifique ou non, inductible ou non, forte ou faible. Il peut s'agir en particulier de promoteurs ubiquitaires (promoteur des gènes HPRT, PGK, α-actine, tubuline, etc), de promoteurs des filaments intermédiaires (promoteur des gènes GFAP, desmine, vimentine, neurofilaments, kératine, etc), de promoteurs de gènes thérapeutiques (par exemple le promoteur des gènes MDR, CFTR, Facteur VIII, ApoAI, etc), de promoteurs spécifiques de tissus (promoteur du gène pyruvate kinase, villine, protéine intestinale de liaison des acides gras, α-actine du muscle lisse, etc) ou encore de promoteurs répondant à un stimulus (récepteur des hormones stéroïdes, récepteur de l'acide rétinoique, etc). De même, il peut s'agir de séquences promotrices issues du génome d'un virus, tel que par exemple les promoteurs des gènes E1A et MLP d'adénovirus, le promoteur précoce du CMV, ou encore le promoteur du LTR du RSV, etc. En outre, ces régions promotrices peuvent être modifiées par addition de séquences d'activation, de régulation, ou permettant une expression tissu-spécifique ou majoritaire.

Par ailleurs, le gène d'intérêt peut également comporter une séquence signal dirigeant le produit synthétisé dans les voies de sécrétion de la cellule cible. Cette séquence signal peut être la séquence signal naturelle du produit synthétisé, mais il peut également s'agir de toute autre séquence signal fonctionnelle, ou d'une séquence signal artificielle.

Selon le gène d'intérêt, les molécules d'ADN de l'invention peuvent être utilisées pour le traitement ou la prévention de nombreuses pathologies, incluant les maladies génétiques (dystrophie, fibrose cystique, etc), les maladies neurodégénératives (alzheimer, parkinson, ALS, etc), les cancers, les pathologies liées aux désordres de la coagulation ou aux dyslipoprotéinémies, les pathologies liées aux infections virales (hépatites, SIDA, etc), ou dans les domaines agronomique et vétérinaire, etc.

Par ailleurs, la présente invention concerne également l'utilisation des molécules d'ADN à réplication conditionnelle pour la production de protéines recombinantes. Les bactéries peuvent être utilisées pour produire des protéines d'origines diverses, eucaryotes ou procaryotes. Parmi les bactéries, E. coli constitue l'organisme de choix pour l'expression de gènes hétérologues en raison de sa manipulation aisée, du nombre important de systèmes d'expression disponibles et des quantités importantes de protéines que l'on peut obtenir. Il est entendu que le système de l'invention est utilisable dans d'autres organismes, le tropisme étant déterminé par la nature de l'origine de réplication, comme indiqué ci-avant. Pour cette utilisation, la séquence nucléique d'intérêt comprend une région codante sous le contrôle de signaux d'expression appropriés à l'hôte choisi, en particulier un hôte procaryote. Il peut s'agir par exemple des promoteurs Plac, Ptrp, PT7, Ptrc, Ptac, PL, PR, séquence Shine-Dalgamo, etc (cet ensemble constitue la cassette d'expression). La séquence d'acide nucléique d'intérêt peut être toute séquence codant pour une protéine présentant un intérêt dans les domaines de la pharmacie, de l'agro-alimentaire, de la chimie ou de l'agrochimie. Il peut s'agir d'un gène de structure, d'une séquence d'ADN complémentaire, d'une séquence synthétique ou semi-synthétique, etc.

La cassette d'expression peut être introduite sur le vecteur à réplication conditionnelle sujet de l'invention, constituant ainsi un vecteur à réplication conditionnelle permettant l'expression de protéines d'intérêt chez E. Coli. Ce vecteur présente plusieurs avantages: pas d'utilisation d'antibiotique pour le sélectionner chez la bactérie (moindre coût, pas de nécessité d'étude quant à la présence dans le produit fini d'antibiotique ou de produits dérivés potentiellement toxiques), probabilité pratiquement nulle de dissémination du plasmide dans la nature (origine de réplication conditionnelle), fermentation possible en milieu totalement défini. Les exemples présentés montrent les propriétés avantageuses de ces vecteurs conditionnels pour la production de protéines recombinantes.

La présente invention sera plus complètement décrite à l'aide des exemples qui suivent, qui doivent être considérés comme illustratifs et non limitatifs.

### Légende des Figures :

Figure 1: Organisation fonctionnelle de la région de R6K impliquée dans la réplication.
Figure 2: Organisation des domaines fonctionnelles de la protéine π du plasmide R6k.
Figure 3: Représentation du protocole d'introduction du gène pir dans le génome d'E.coli XAC1.
Figure 4: Schéma de contruction des vecteurs pXL2666, 2730 et 2754.
Figure 5: Construction du pXL2774.
Figure 6: Cinétique de croissance et de production en fermenteur de 2L.
Figure 7: Cinétique de croissance et de production en fermenteur de 800L.
Figure 8: Construction du pXL3056.

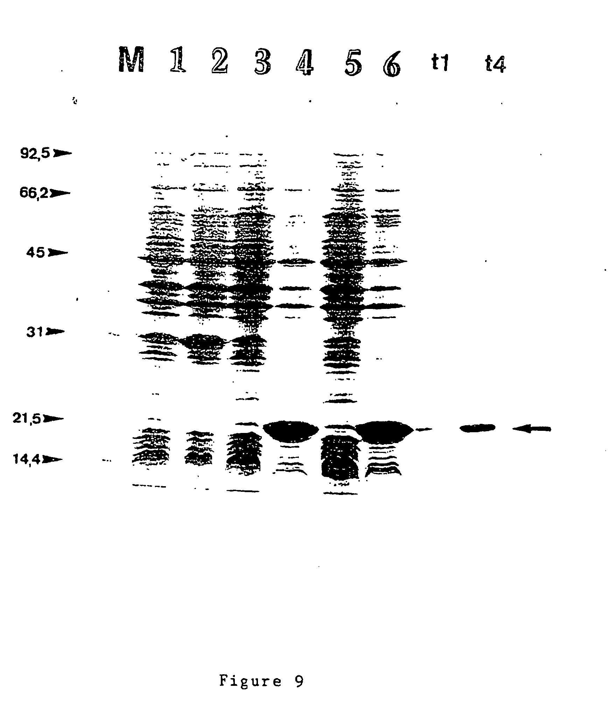

Figure 9 : Visualisation de la protéine aFGF produite par E. coli XAC-1pir-11 (pXL305b+PT7po123) après induction. Les extraits cellulaires totaux dénaturés sont déposés sur gel de polyacrylamide 12,5%-SDS. M: marqueur de masse moléculaire (Biorad, Low range). Chaque bande est identifiée par une flèche et un chiffre qui indique sa masse en kDaltons. 1: XAC-1pir-11 (pXL3056+pUC4K) non induite; 2: XAC-1pir-116 (pXL3056+pUC4K) induite à 42°C; 3: XAC-1pir-116 (pXL3056+PT7po123) clone 1, non induite; 4: XAC-1pir-116 (pXL3056+PT7po123) clone 1, induite à 42°C; 5: XAC-1pir-116 (pXL3056+PT7po123) clone 2, non induite; 6: XAC-1pir-116 (pXL3056+PT7po123) clone 2, induite à 42°C; t1: 1µg de aFGF purifié; t4: 4µg de aFGF purifié.
Figure 10 : Schéma de contruction des vecteurs pXL3029 et pXL3030.

### I - MATERIELS ET METHODES

### A) Matériels

### 1) Milieux de culture

Les milieux complets LB, 2XTY et SOC, le milieu minimum M9 (Maniatis et al., 1989) ont été utilisés. Les milieux gélosés ont été obtenus par addition de 15 g d'agar Difco. De plus, si nécessaire, ces milieux ont été supplémentés avec des antibiotiques, ampicilline ou kanamycine, aux concentrations respectives de 100 mg/l et de 50 mg/l. Les substrats chromogènes X-Gal et X-Gluc ont été utilisés à la concentration de 40 mg/l.

### 2) Souches d'E. coli, plasmides et bactériophages

Les souches d'E. coli, les plasmides et les bactériophages utilisés sont identifiés respectivement dans les exemples ci-après.

### B) Méthodes

### 1) Manipulation de l'ADN

L'isolement d'ADN bactérien (plasmidique, génomique) et phagique (forme replicative de M13), les digestions par les endonucléases de restriction, les ligatures de fragments d'ADN, l'électrophorése en gel d'agarose (en tampon TBE) et autres techniques standard ont été réalisées suivant les recommandations des fournisseurs, pour l'utilisation d'enzymes, ou en se conformant aux protocoles décrits dans "Molecular Cloning: a Laboratory Manual" (Maniatis et al., 1989).

Les marqueurs de taille d'ADN utilisés lors des électrophorèses sont les suivants : échelle 1 kpb (BRL) pour les fragments linéaires et le marqueur d'ADN surenroulé (Stratagène) pour les plasmides non digérés.

Le séquençage a été réalisé selon la technique de Sanger (Sanger et al., 1977) adaptée à la méthode automatisée utilisant des didéoxynucléotides fluorescents et la Taq ADN polymérase (PRISM Ready Reaction DyeDideoxy Terminator Cycle Sequencing Kit, Applied Biosystems).

Les oligodésoxynucléotides utilisés (désignés par "seq+n°", voir ci-dessous) ont été synthétisés sur le synthétiseur "Applied Biosystems 394 DNA/RNA Synthesizer" par la méthode des phosphoramidites, utilisant des groupements protecteurs ß-cyanoéthyles (Sinha et al., 1984). Après synthèse, les groupements protecteurs sont éliminés par traitement à l'ammoniaque. Deux précipitations au butanol permettent de purifier et de concentrer l'oligonucléotide (Sawadogo et al., 1991).

### Sequence des oligonucléotides utilisés pour l'amplification par PCR :

Les réactions de PCR (Saïki et al., 1985) ont été réalisées dans les conditions suivantes, dans un volume total de 100 µl. Le mélange réactionnel comprend 150 ng d'ADN génomique de la souche à étudier, 1 µg de chacun des 2 oligonucléotides-amorces (24-mer), 10 µl de tampon 10XPCR, dont la composition est la suivante "500 mM KCI, 0,1 % gélatine, 20 mM MgCl₂, 100 mM Tris-HCl pH 7,5", et 2,5 unités de Taq ADN polymérase (Amplitaq Perkin-Elmer). Les conditions de PCR, sur l'appareil Perkin-Elmer Cetus DNA Thermal Cycler, sont les suivantes: 2 min à 91°C, 30 cycles successifs de dénaturation (1 min à 91°C), d'hybridation (2 min à 42°C) et d'élongation (3 min à 72°C), et enfin 5 min à 72°C. Les produits ainsi obtenus, digérés ou non par une enzyme de restriction, sont analysés par électrophorèse sur gel d'agarose.

L'analyse de différentes espèces plasmidiques par les ADN topo-isomérases a été réalisée selon le protocole suivant: Les enzymes, purifiées dans son laboratoire, sont incubées pendant 1 heure à 37°C. Les mélanges réactionnels (volume total: 40 µl) ont la composition suivante : 150 ng de plasmide, 300 ng d' ADN topo-isomérase I, ou 150 ng d'ADN gyrase d' E. coli, ou 160 ng d' ADN topo-isomérase IV de S. aureus et 20 µl de tampon spécifique de chaque enzyme. La composition de ces tampons est indiquée ci-dessous :
pour l'ADN topo-isomérase 1: 50 mM Tris-HCl pH 7.7, 40 mM KCI,
   1 mM DTT,100 µg/ml SAB, 3 mM MgCl₂,1 mM EDTA;
pour l'ADN topo-isomérase IV : 60 mM Tris-HCl pH 7.7, 6 mM MgCl₂,
   10 mM DTT, 100 µg/ml SAB, 1.5 mM ATP,
   350 mM glutamate de potassium;
pour l'ADN gyrase : 50 mM Tris-HCl pH 7.7, 5 mM MgCl₂,
   1.5 mM ATP, 5 mM DTT, 100 µg/ml SAB, 20 mM KCl.

### 2) Transformation d'E. coli

Elle a été réalisée en routine selon la méthode TSB (Transformation and Storage Buffer) décrite par Chung et Miller (1988). Pour une souche comme TG1 (Gibson et al. 1984), l'efficacité de transformation obtenue est de l'ordre de 10⁵-10⁶ transformants par µg de pUC4K (Vieira et Messing; 1982). Lorsqu'une efficacité de transformation plus élevée était nécessaire, les bactéries ont été transformées par électroporation selon le protocole préconisé par le fabricant de l'électroporateur (Biorad). Cette méthode permet d'atteindre des efficacités de 10⁸ à 10¹⁰ transformants par µg de pUC4K.

### 3) Transfection cellulaire médiée par un lipofectant cationique

Les cellules utilisées sont des fibroblastes murins NIH 3T3, ensemencées le jour précédent en plaques 24 puits, à une densité de 50 000 cellules par puits. Le milieu de culture utilisé est le milieu DMEM, contenant 4,5 g/l de glucose, complété avec 10 % de sérum de veau foetal et 1 % des solutions de glutamine 200 mM et d'antibiotiques (streptomycine 5.10³ u/ml, pénicilline 5.10³ µg/ml) (Gibco). L'ADN plasmidique (1 µg dans 25µl de NaCl 9 ‰) est mélangé, volume à volume, à une suspension de lipofectant. Quatre rapports "charges du lipofectant / charges de l'ADN" sont testés : 0, 3, 6 et 9. Ces rapports sont calculés en considérant que 1 µg d'ADN plasmidique porte 3,1 nmoles de charges négatives et que le lipofectant comporte 3 charges positives par molécule. Après un contact de 10 minutes permettant la formation du complexe ADN/lipide, 50 µl de mélange ADN-lipofectant est introduit sur les cellules en milieu de culture sans sérum (500 µl). Les cellules ont été préalablement rincées 2 fois avec ce même milieu. L'inhibition de la transfection par le sérum est ainsi évitée. Après incubation (2 heures à 37°C dans l'incubateur à CO₂), le milieu est additionné de 10 % de sérum de veau foetal. Les cellules sont ensuite remises à incuber pendant 24 heures.

### 4) Mesure de l'activité luciférase de cellules eucaryotes

Elle est effectuée 24 heures après la transfection. La luciférase catalyse l'oxydation de la luciférine, en présence d'ATP, de Mg²⁺ et d'O₂ avec production concomitante d'un photon. L'émission totale de lumière, mesurée par un luminomètre, est proportionnelle à l'activité luciférase de l'échantillon. Les réactifs utilisés sont fournis par Promega (Luciferase assay system) et utilisés selon le protocole conseillé. Après lyse des cellules, la fraction insoluble de chaque extrait est éliminée par centrifugation. Le dosage est effectué sur 5 µl de surnageant, dilué ou non dans le tampon de lyse des cellules.

### 3) Mesure de la concentration protéique des extraits cellulaires

Elle est effectuée selon la méthode BCA (Pierce) utilisant l'acide bicinchoninique (Wiechelman et aL, 1988). La gamme- étalon de SAB est réalisée dans le tampon de lyse (cf III-B-4). Les échantillons à doser et ceux de la gamme sont prétraités, volume à volume, avec de l'iodoacétamide 0,1 M/tampon Tris 0,1 M pH. 8,2. pendant 1 heure à 37°C. Ce traitement permet d'éviter l'interférence, lors du dosage, de l'agent réducteur (DTT) présent dans le tampon de lyse. La lecture du dosage s'effectue à 562 nm.

### EXEMPLE 1:

### Construction des souches-hôtes XAC-1pir et pir-116 par recombinaison homologue.

La souche mise en oeuvre est la souche E. coli XAC-1 (Normanly et al; 1980. Avantageusement, le gène argE de cette souche comporte une mutation Glutamine-53 (CAG) en codon ambre (TAG) (Meinnel et aL, 1992). Le gène argE appartient à l'opéron divergent argECBH et code pour une enzyme de biosynthèse de l'arginine, la N-acétylomithinase. XAC-1 ne peut donc synthétiser l'arginine et, en conséquence, croître en milieu minimum. Cette auxotrophie sera levée si la souche abrite un plasmide permettant l'expression d'un ARNt suppresseur. Il sera donc possible, par culture en milieu minimum, de sélectionner les bactéries qui portent un tel plasmide. Pour y permettre la réplication des plasmides dérivés de R6K, il a été nécessaire d'introduire; par recombinaison homologue, le gène pir dans le génome de XAC-1. Le gène pir (sauvage ou muté) est introduit au locus uidA par échange entre le gène uidA sauvage et une copie interrompue par le gène pir (ou pir-116). Le gène uidA code pour une ß-glucuronidase, enzyme d'hydrolyse des ß-glucuronides. Ce gène peut être inactivé sans problème puisqu'il n'est pas essentiel à la croissance dans les milieux synthétiques classiques, dans lesquels les β-glucuronides ne sont pas utilisés. De plus, l'activité β-glucuronidase peut être suivie grâce à un substrat chromogène, le X-Gluc, dont l'hydrolyse libère un pigment bleu.

### 1) Construction d'un vecteur-suicide portant la cassette "Km^{R}-uidA::pir (ou pir-116)

Nous avons utilisé une stratégie impliquant un seul hôte bactérien et minimisant les modifications du génome de la souche d'intérêt. Le phage M13mp10 (Messing et Vieira; 1982) a été utilisé comme vecteur-suicide (Blum *et al*, 1989). Une mutation ambre dans le gène II, essentiel pour la réplication, réduit le spectre d'hôte de ce M13 aux souches, telle TG1 *(supE),* qui produisent un ARNt suppresseur d'ambre; il ne pourra donc se répliquer dans les souches d' *E. coli.sup*+, comme XAC-1.

Les cassettes *Bam*HI de 3,8 kpb, contenant le gène de résistance à la kanamycine de Tn*5* et *_uidA::pir* ou *pir-116*, ont été purifiée, respectivement, à partir de M13wm34 et 33 ( Metcalf et al; 1994). Elles ont été clonées dans M13mp10 linéarisé par *Bam*HI. Les clones recombinants ont été sélectionnés par étalement sur milieu gélosé LB + Km, après électroporation dans TG1 des mélanges de ligature. La conformité des clones obtenus a été montrée par analyse du profil de restriction et par séquençage de la région correspondant à la mutation *pir-116.*

### 2) Introduction par recombinaison homologue des gènes pir ou pir-116 dans le génome d'E. coli XAC-1

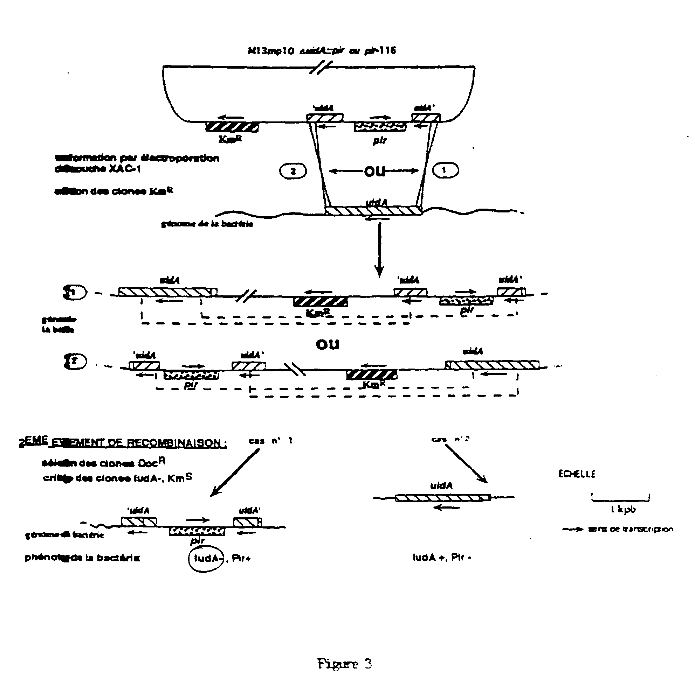

La stratégie adoptée et les différents événements impliqués sont présentés dans la figure 3.

### a) Premier événement de recombinaison

La souche XAC-1 a été transformée par électroporation avec 10, 100 ou 2000 ng de chaque RF (mp10-_*uid4::pir* ou *pir-116).* Un tiers de chaque mélange d'expression a été étalé sur boites LB contenant de la kanamycine et incubé la nuit à 37°C. Les phages mp10-_*uidA:: pir* ou *pir-116* ne peuvent se répliquer dans la souche XAC-1 (*sup*+). Le marqueur Km^{R} ne peut donc se maintenir que par intégration dans le génome de la bactérie, via une recombinaison homologue avec la copie sauvage du gène *uidA.* Les résultats des électroporations de XAC-1 sont présentés dans le tableau 1. L'efficacité de transformation obtenue était de 4.10⁹ transformants par µg de pUC4K.

**TABLEAU 1**

| **CONSTRUCTION** | **Nombres de colonies obtenues avec les quantité d'ADN transformé** | | |
|---|---|---|---|
| | **10 ng** | **100 ng** | **2000 ng** |
| M13mp10*-_uidA: pir* | 1 | 41 | 146 |
| M13mp10-_*uidA::pir-116* | 0 | 16 | 124 |

Dans les conditions testées, le nombre d'intégrants croît de façon non linéaire avec la quantité d'ADN. Connaissant l'efficacité de transformation et la taille des RF (11,7 kpb), on peut avoir une idée approximative du taux de recombinaison. En considérant le point à 100 ng, on obtient une fréquence de recombinaison de l'ordre de 10⁻⁶.

### b) Deuxième événement de recombinaison

Le deuxième événement de recombinaison sera ensuite sélectionné par la résistance des souches au désoxycholate (Doc^{R}).
Pour ce faire, 5 intégrants de chaque construction ont été mis en culture en milieu 2XTY additionné de 0,2 % de désoxycholate de sodium. Deux populations distinctes sont apparues. Certains clones donnent un trouble bien visible après environ 8 heures à 37°C (deux clones pour la construction *pir* et trois pour la construction *pir-116).* Les autres clones ont donné une culture dense seulement après la nuit à 37°C. Ils étaient quasiment tous Km^{S}, comme attendu. Pour chacun des électroporants étudiés, 50 descendants Km^{S} ont été striés sur LB additionné de X-Gluc. Après 48 heures à 37°C, les clones UidA⁺ étaient bleu pâle alors que ceux qui avaient subi un remplacement d'allèle (cas n°1, figure 3), sont restés blancs sur ce milieu (UidA⁻). Le tableau 2 résume l'analyse des phénotypique des recombinants doubles obtenus.

De 18 à 30 % des doubles recombinants ont subi un remplacement d'allèle.

**TABLEAU 2**

| **souche** | **nombre de Km**^{**s**} **parmi les Doc**^{**R**} | **pourcentage de UidA**^{**-**} **parmi les Km**^{**s**} |
|---|---|---|
| XAC-1 *pir-2* | 50/50 | 18 |
| XAC- 1 *pir-3* | 50/50 | 24 |
| XAC -1 *pir-4* | 50/50 | 34 |
| XAC-1 *pir-116*-1 | 50/50 | 32 |
| XAC *pir-116*-4 | 35/50 | 30 |

### 3) Contrôle du caractère Pir+ des souches obtenues par recombinaison

Afin de s'assurer du caractère Pir+ des souches obtenues par double recombinaison, nous avons transformé trois clones de chaque construction par pBW30 (Metcalf *et al.,* 1994). L'obtention de transformants pour toutes les souches testées a permis de montrer la fonctionnalité des gènes *pir* et *pir-116* intégrés dans le génome de XAC-1. Dans les mêmes conditions, aucun transformant n'est obtenu avec la souche parentale XAC-1. Nous avons poursuivi l'étude de 2 clones XAC-1*pir* (B et C) et 2 clones XAC*-*1*pir-116* (E et D).

### 4) Contrôle par amplification par PCR des souches obtenues par recombinaison

Pour confirmer le remplacement d'allèle, nous avons contrôlé par amplification par PCR les régions génomiques de part et d'autre du locus *uidA.* Chaque couple d'oligonucléotides est constitué d'un oligonucléotide correspondant à une région interne de *pir* et d'un deuxième oligonucléotide correspondant à une région, proche de *uidA* chromosomique, mais non comprise dans le fragment qui a servi à la recombinaison. La séquence de ce dernier oligonucléotide a été déterminée grâce à la séquence ECOUIDAA de Genbank (numéro d'accès : M14641). Nous avons ainsi pu vérifier le positionnement exact du gène *pir* dans le génome de la bactérie. La nature des fragments amplifiés, dont la taille est conforme à celle que l'on pouvait prévoir, a été confirmée par digestion par *Mlu*I.

### EXEMPLE 2

### Construction de vecteurs plasmidiques dérivés de R6K portant le marqueur de sélection sup Phe

Nous avons construit des vecteurs comportant l'ori γ de R6K et le gène de résistance à la kanamycine (pXL2666). L'observation de multimères de pXL2666 dans la souche BW19610 *(pir-116)* 5 (Metcalf et al; 1993 nous a amené à étudier l'effet du fragment *cer* de ColE1 sur ce phénomène. Nous avons ensuite introduit sur le vecteur ori γ -Km^{R}-cer (pXL2730) la cassette d'expression de l'ARNt suppresseur Phénylalanine (*sup* Phe). Ce vecteur, pXL2760, sert de base à la construction de vecteurs utilisables en thérapie génique.

### 1) Construction et analyse des vecteurs comportant l' ori γ de R6K et le gène de résistance à la kanamycine

### a) Constructions

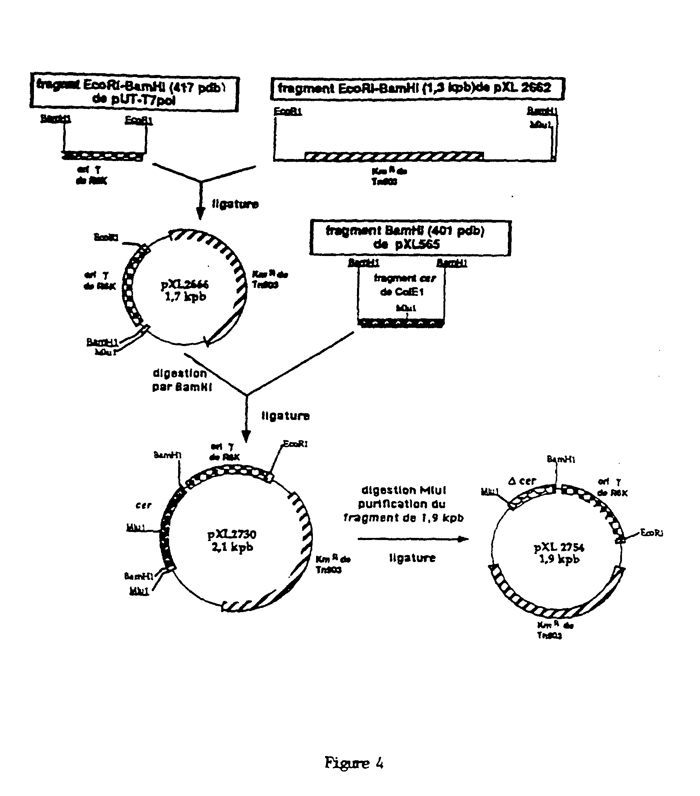

Dans le premier plasmide construit, pXL2666, le gène de résistance à la kanarnycine provient de pUC4K (Vieira et messing; 1982) et l'origine de réplication, contenue dans un fragment *Eco*RI-*Bam*HI de 417 pdb, provient du vecteur-suicide pUT-T7pol (Herrero et al; 1990) (figure 4). Le transfert de pXL2666 dans les souches BW19094 et 19610 (Metcalf et al; 1994) a permis de montrer que la quantité de plasmide est bien augmentée dans une souche *pir-116,* par rapport au même plasmide dans une souche *pir.* Toutefois, l'analyse par électrophorèse des plasmides non digérés montre que cette augmentation va de pair avec l'apparition de quelques formes multimériques. Vraisemblablement, ce phénomène est lié à une recombinaison intermoléculaire entre les multiples copies du plasmide. Aussi avons nous construit pXL2730 en clonant sur pXL2666 le fragment *cer* du plasmide naturel d'E. coli Co1E1, dont il avait été montré qu'il permettait, en *cis,* la résolution des dimères de plasmides (Summers and Sherrat, 1984). Le fragment utilisé correspond à un fragment *Hpa*II de 382 pdb de ColE1 (Leung *et al.,* 1985). Il contient un site de recombinaison intermoléculaise spécifique; il implique uniquement, pour être fonctionnel, des protéines de l'hôte dont les recombinases XerC et XerD, et les facteurs accessoires ArgR et PepA (Stirling *et al.,* 1988, 1989; Colloms *et al.,* 1990). Pour s'assurer que les effets observés sont bien dus au fragment *cer,* nous avons aussi construit le plasmide contrôle pXL2754 dans lequel le fragment *cer* est délété de 165 pdb. Cette délétion a été montrée comme abolissant l'action de *cer* sur la résolution des multimères (Leung *et al.,* 1985). Les différentes étapes de clonage conduisant à la construction de ces plasmides sont présentées sur la figure 4.

### b) Analyse quantitative et qualitative des espèces plasmidiques

### •analyse par électrophorèse en gel d'agarose

L'analyse par électrophorèse des différents plasmides construits a permis la mise en évidence de diverses espèces plasmidiques, variables suivant les souches utilisées. La taille des plasmides non digérés a été évaluée par rapport au marqueur d'ADN surenroulé. Dans la souche *pir* (BW19094), les plasmides pXL2666, 2754 et 2730 sont pratiquement entièrement sous forme monomérique. Les bandes au dessus de chaque bande principale correspondent à différents topo-isomères, légèrement moins surenroulés, comme le confirme le profil observé après action de l'ADN gyrase avec pXL2730.

Dans le cas de la souche pir-116 (BW19610), les profils sont différents : on observe avec les plasmides pXL2666 et 2754 différentes espèces allant du monomère aux multimères (2, 3 ou 4 unités), la forme majoritaire étant le dimère. Après digestion par EcoRI, on ne retrouve que l'ADN plasmidique linéaire; ces espèces plasmidiques correspondent soit à des multimères de plasmides, soit à des topo-isomères divers. Toutefois, la taille des formes déterminée d'après le marqueur d'ADN surenroulé étant un produit entier de celle du plasmide monomère, il est fort probable qu'il s'agisse de multimères. La formation des multimères est très probablement imputable à la mutation *pir-116,* bien que les deux souches BW19094 et BW19610 ne soient pas strictement isogéniques (BW19610 est *recA*). Le profil obtenu avec pXL2730 est différent : bien que des formes multimériques soient encore visibles, la forme majoritaire est la forme monomérique. Le fragment *cer* peut donc faciliter la résolution des multimères de plasmide que nous avons construit et ceci de façon indépendante de *recA,* dans BW19610.

### •analyse après traitement par les ADN topo-isomérases

Afin d'écarter l'hypothèse selon laquelle les formes observées dans les souches portant l'allèle *pir-116* seraient des topo-isomères particuliers, chaque préparation de plasmide a été soumise à l'action d'ADN topo-isomérases. Les activités des différentes enzymes, dans les conditions expérimentales sont les suivantes : relâchement de l'ADN pour l'ADN topo-isomérase 1 d'*E*. *coli,* surenroulement négatif de l'ADN relâché pour l'ADN gyrase d'*E. coli,* et désenchevêtrement des ADN entrelacés et relâchement de l'ADN surenroulé par l'ADN topo-isomérase IV de *S. aureus.* L'action de l'ADN topo-isomérase IV a permis de montrer que les formes plasmidiques de haut poids moléculaire ne résultaient pas de l'enchevêtrement de plusieurs molécules de plasmides; dans ce cas, elles auraient alors été converties en l'espèce monomérique. La fonctionnalité de l'enzyme a bien sûr été contrôlée sur une préparation d'ADN de kinétoplastes, composée de molécules d'ADN enchevêtrées (non montré). L'activité de relâchement est aussi visible puisqu' on obtient des espèces migrant moins que dans les témoins non traités. L'action de l'ADN gyrase a permis de convertir les topo-isomères légèrement relâchés en l'espèce la plus surenroulée extraite de la bactérie (monomère ou dimère principalement). De plus, elle a permis de vérifié que les ADN préparés sont majoritairement sous forme surenroulée. Les échantillons ainsi traités permettent de confirmer les résultats précédents quant aux espèces majoritaires pour chaque construction. L'ADN topo-isomérase I a bien relâché l'ADN mais de façon partielle. Ceci pourrait être dû au fait que les plasmides étudiés ne comportent que peu de zones simples brins, sur lesquelles cette enzyme se lie préférentiellement (Roca, 1995).

### 2) Introduction du marqueur de sélection sup Phe sur pXL2730

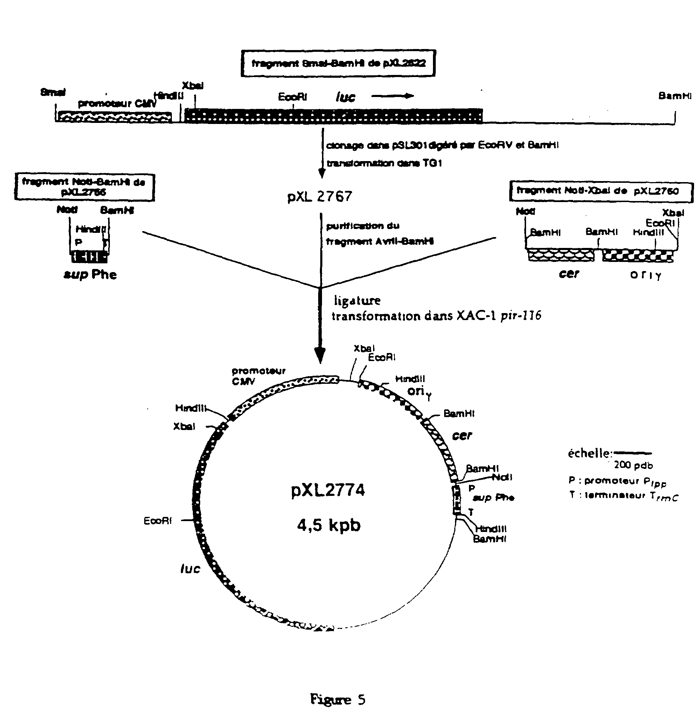

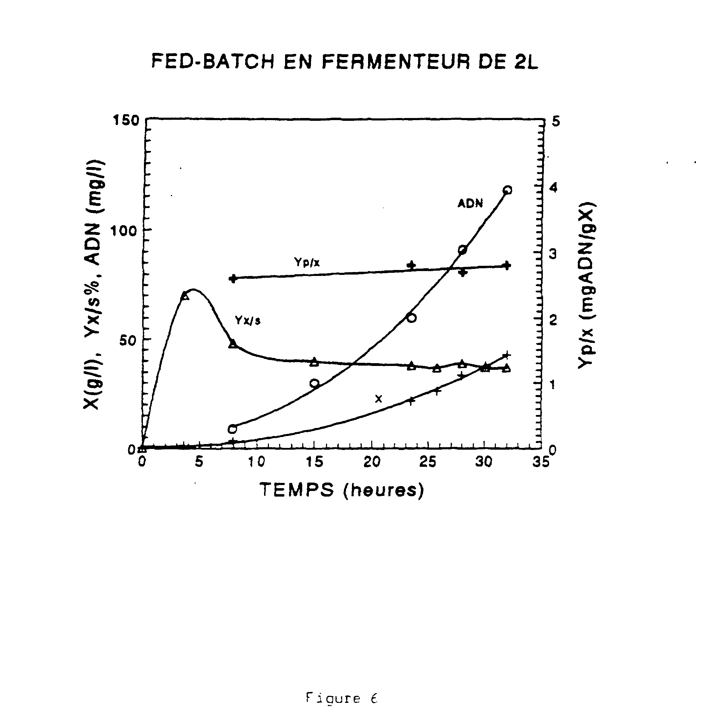

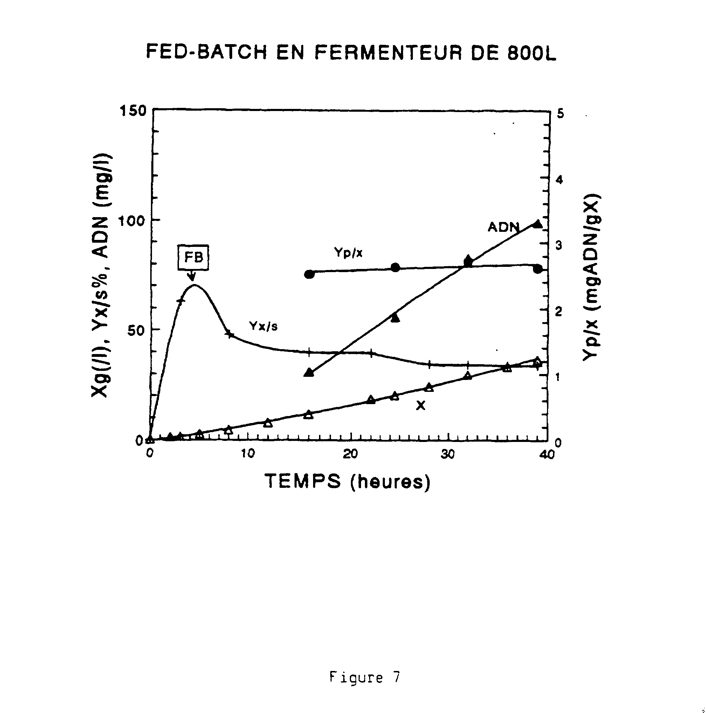

Nous avons utilisés la cassette d'expression du gène d'ARNt suppresseur synthétiques (Phe) (Kleina *et al.,* 1990). Celui-ci introduit dans la chaîne polypeptidique en formation une Phénylalanine en réponse à un codon TAG. De plus, il permet la production dans XAC-1 d'une protéine ArgE suffisamment active pour permettre une bonne croissance en milieu carencé en arginine. Sur le plasmide pCT-2-F (Normanly et al; 1986), *sup* Phe est exprimé de manière constitutive à partir d'un promoteur synthétique dérivé de la séquence du promoteur du gène *lpp* d' *E. coli,* P_{*lpp*}*.* En aval de ce gène, l'arrêt de transcription est assuré par le terminateur synthétique de l'opéron *rrnC* d'*E. coli,* T_{*rmC*} (Normanly *et al.,* 1986). Les différentes étapes de clonage sont indiquées sur la figure 5.

Les différents sous-clonages ont été réalisés dans XAC-1. La fonctionnalité de la cassette d'expression de l'ARNt suppresseur est ainsi contrôlée grâce à l'activité β-galactosidase de cette souche qui n'existe que s'il y a suppression du codon ambre du gène lacZᵤ₁₁₈ₐₘ. La dernière étape consiste en l'introduction de la cassette d'expression de *sup* Phe sur pXL2730. Les résultats obtenus avec le fragment *cer* (B-1-*b*) nous ont fait choisir ce plasmide plutôt que pXL2666. Nous avons conservé le gène de résistance à la kanamycine pour des facilités de clonage ultérieur, notamment pour disposer d'un criblage supplémentaire lors du clonage final (perte de Km^{R}).

### EXEMPLE 3:

### Validation du vecteur plasmidique pour des applications en thérapie génique par transfection de fibroblastes murins.

### 1) Construction du vecteur rapporteur pXL2774

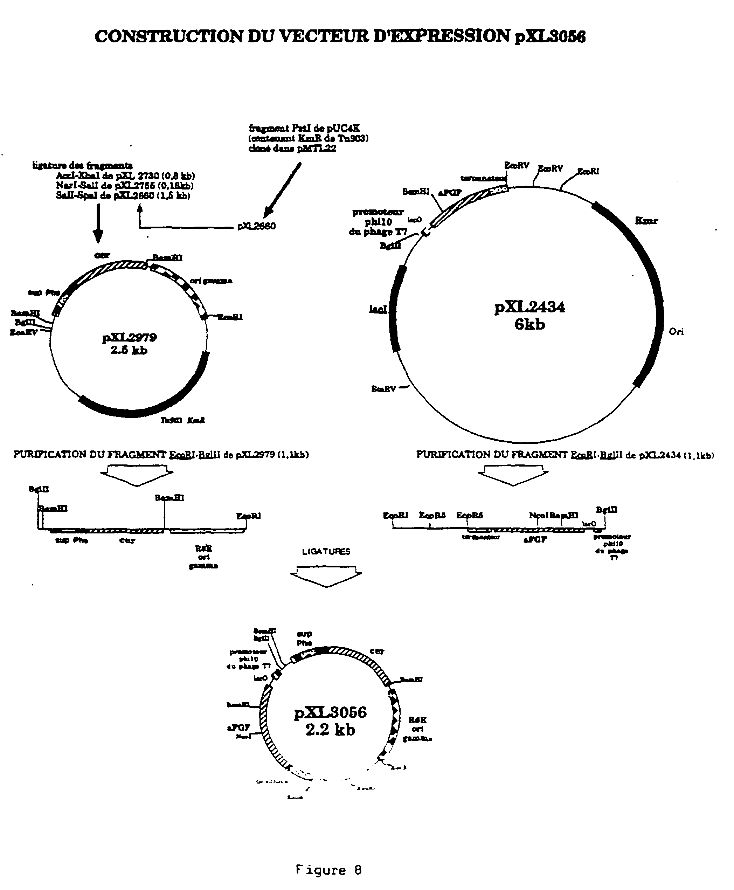

Afin de tester la validité en thérapie génique du système de production d'ADN plasmidique, nous avons introduit sur pXL2760 un gène rapporteur, utilisable dans les cellules eucaryotes. Nous avons utilisé le gène *luc* codant pour la luciférase de *Photinus pyralis* car le test de mesure de bioluminescence est très sensible, linéaire sur une large gamme et le bruit de fond dû à l'activité endogène des cellules eucaryotes est très faible. Le gène *luc* est sous contrôle des séquences promotrices-amplificatrices d'un gène précoce du cytomégalovirus humain (promoteur CMV) qui permet une expression à taux élevé. En 3' de *luc* se trouve une région non traduite, provenant du virus SV40, qui contient le signal de polyadénylation (poly(A)+). Après un clonage intermédiaire permettant d'augmenter le nombre de sites de restriction disponibles, la cassette "promoteur CMV-*luc*-poly(A)+" est introduite sur le vecteur minimal ori γ-*cer-sup* Phe (pXU760) à la place du marqueur Km^{R}. Le plasmide résultant a été appelé pXL2774. La figure 6 rassemble les diverses étapes de clonage. Les mélanges de ligature ont été transformés par électroporation dans XAC*-*1*pir-116.* L'incubation permettant aux bactéries l'expression des marqueurs de sélection s'effectue en milieu riche (milieu SOC); il a donc été nécessaire de laver les cellules deux fois avec du milieu M9 avant l'étalement. Ceci a permis d'éliminer le milieu résiduel qui aurait entraîné un bruit de fond de culture sur milieu minimum.

Le milieu choisi pour étaler les cellules électroporées est le milieu minimum M9, qui permet de sélectionner les bactéries exprimant un ARNt suppresseur et donc la présence de nos plasmides. L'addition de X-Gal permet, par la coloration bleue, de visualiser l'expression de l'ARNt suppresseur. Les boites sont analysées après environ 20 heures à 37°C. L'absence de colonies sur le témoin sans ADN nous assure que la sélection est correcte, même avec des ensemencements denses. Tous les clones examinés par restriction (8) portent bien un plasmide, correspondant au profil attendu. Le plasmide ainsi construit, pXL2774, a été préparé à partir d'un clone cultivé dans un litre de milieu liquide M9 (environ 18 heures à 37°C), par une technique faisant appel entre autre à un échange d'ions (kit Promega, MegaPreps). La quantité d'ADN recueillie est de 2 mg.

### 2) Analyse du vecteur rapporteur pXL2774 transfecté dans les cellules de mammifères

La capacité de pXL2774 à transfecter les cellules eucaryotes et à permettre l'expression de la luciférase est évaluée par transfection dans les fibroblastes murins NIH 3T3. Le vecteur choisi comme référence est le plasmide pXL2622 (il s'agit du plasmide pGL2 de Promega dont le promoteur SV40 a été remplacé par le promoteur CMV) qui porte la même cassette d'expression de la luciférase que pXL2774, mais sur un réplicon différent. C'est un dérivé de ColE1, de 6,2 kpb, qui porte le gène de résistance à l'ampicilline. Ce plasmide nous sert de témoin. Les activités luciférase (exprimée en RLU, ou unités relatives de luminescence) sont indiquées dans le tableau 3.

Les résultats les meilleurs ont été obtenus avec un rapport"charges lipofectant/charges ADN" de 6 ; dans ces conditions, pXL2622 et 2774 semblent équivalents.

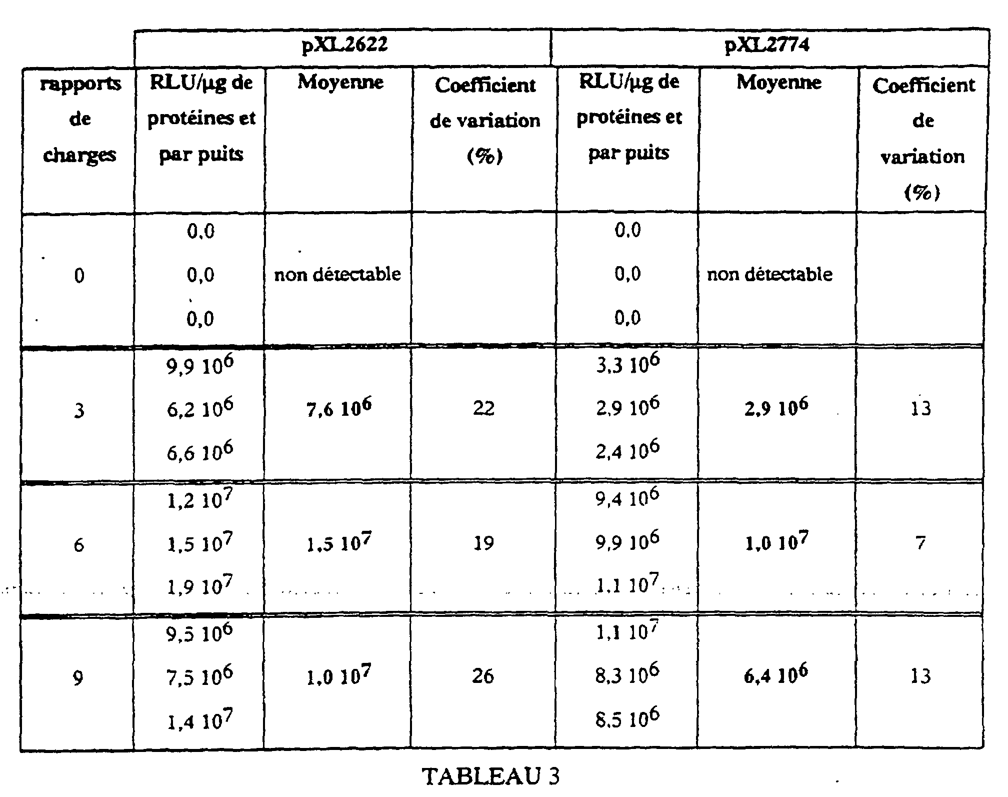

### EXEMPLE 4

### Vérification du caractère de vecteur suicide chez E. coli des plasmides pCOR

Le caractère non réplicatif des plasmides dérivés de R6K type pCOR a été vérifié par une expérience d'électroporation dans E. coLi JM109 (Yanisch-Perron et al., 1985) des plasmides pUC4K (ori CoIEI-KmR,(Vieira et Messing, 1982)) et pXL2730 (ori gamma de R6K-KmR. voir exemple 2). L'électroporateur utilisé est le Gene Pulser Biorad et les cellules JM109 électrocompétentes sont préparées et utilisées selon le protocole du fabricant (Bacterial électro-transformation and pulse controller instruction manual. catalog number 165-2098).

Les cellules électrotransformées ont été étalées sur milieu LB additionné de kanamycine (50mg/l) et incubées la nuit à 37°C. Les résultats obtenus sont présentés ci-dessous.

Résultats:

| Plasmide | quantité transformée (ng) | nombre de transformants | Efficacité (nombre de transformants/ng de plasmide) |
|---|---|---|---|
| pUC4K | 0,01 | >>2000 | >2105 |
| pXL2730 | 5 | 0 | 0 |

Ces résultats montrent qu'il y a au minimum 5 log de différence entre l'efficacité de transformation d'un dérivé de Co1E1 (pUC4K) par rapport à un dérivé de R6K (pXL2730) dans une souche n'exprimant pas le gène pir. Dans une souche pir+ comme XAC-1pir-116 l'efficacité d'électrotransformation de plasmides dérivés de R6K atteint ou dépasse classiquement les 108 transformants / par µg de plasmide.

### EXEMPLE 5:

### Production d'ADN plasmidique par culture à haute densité de la souche E.coli XAC-1pir-116 (pXL2774) : Procédé de fermentation.

5.1. Souches : Production dans E.coli XAC-1pir-116 (exemple 1), d'un plasmide minimal, pXL2774; Ce plasmide comprend les éléments suivants: ori R6K-cer-tRNAamsupPhe et une cassette d'expression du gène rapporteur luc sous contrôle du promoteur CMV (exemple 3). Il a été développé un procédé à haute productivité pour la production de ce type de plasmides.

### 5.2. Milieux et conditions de culture:

### a) Milieu de croissance:

. Composition du milieu défini utilisé pour les cultures inoculum (g/l):Na2HPO4 6, KH2PO4 3, NaCl 0.5, NH4Cl 1, NH4H2PO4 3, glucose 5, MgSO4,7H20 0.24, CaCl2, 2H2O 0.015, thiamine HCl 0.010
. Composition du milieu complexe utilisé pour les cultures en fed-batch (g/l): KH2PO4 8, K2HPO4 6.3, Na2HPO4 1.7, (NH4)2SO4 0.74, NH4Cl 0.12, extrait de levure 3, glucose 2, MgSO4,7H2 2.4g/l, CaCl2,2H2O 0.015, thiamine 0.010, solution de sels (Fe, Mn, Co, Zn, Mo, Cu, B, Al).
. Composition du milieu défini pour les cultures en fed-batch milieu identique au milieu complexe mais l'extrait de levure est remplacé par 2.5 g/l de NH4Cl.

### b) Conditions de culture en fed-batch:

Des études en fermenteurs de 2 litres (Setric France) contenant Il de milieu ont été réalisées afin de définir les conditions optimales de croissance et de production d'ADN plasmidique. Le fermenteur a été ensemencé avec 80 ml d'une culture inoculum arrivée en début de phase stationnaire de croissance.

Pendant la fermentation, le pH a été contrôlé et ajusté automatiquement entre 6,9 et 7,0 avec de l'ammoniaque 10 % (p/v); la température est maintenue à 37°C ; l'aération a été fixée à 75 1/h ((1.1 vvm) sous une pression de 0.2 bar et l'oxygène dissous a été contrôlé à (40 % de la saturation d'air par rétroaction sur la vitesse d'agitation et, si nécessaire, par enrichissement par de l'oxygène pur.

Tous les paramètres (pH, température, agitation, OD, 02 et CO2 dans les gaz effluents) ont été collectés et calculés en ligne par l'intermédiaire d'une interface HP3852 connectée à un Hewlett-Packard 9000.

La composition de base du milieu d'alimentation est la suivante : source de carbone 50 %, sulfate de magnésium 0.7 %, thiamine 0.02 %; pour le milieu complexe, il a été ajouté de l'extrait de levure à une concentration comprise de préférence entre 5 et 10 %.

Afin d'adapter les conditions de culture aux fermenteurs de 800 litres, des séquences de production comportant deux cultures inoculum successives ont été réalisées, à l'échelle du laboratoire: inoculum I en erlenmeyer agité et inoculum II en fermenteur de 2 litres (cultures en batch), suivie par une culture de production en fed-batch, en fermenteur de 7 litres.

### 5.3. Résultats

Différentes conditions de culture ont été étudiées en milieu complexe, en milieu défini, et à différents taux de croissance. Dans tous les cas, après une culture initiale en batch de la souche bactérienne et consommation de la source de carbone, le milieu d'alimentation a été ajouté au fermenteur grâce à une pompe péristaltique couplée à un profil d'addition pré-programmé. Ce profil a été déduit d'expériences antérieures dans lesquelles le taux d'alimentation avait été asservi soit au taux d'oxygène dissous, soit à un taux de croissance constant.

De plus, de façon à extrapoler sans difficulté les conditions de fermentation de 2 litres à un fermenteur de 8001 sans suroxygénation du milieu, la demande maximale en oxygène en fin de culture a été fixée à 2,5-3 mM/min. Pour cela, le taux de croissance du micro-organisme a été réduit, si nécessaire, par action sur le débit d'alimentation de la charge complémentaire.

Comme le montre le Tableau 4, de très bon résultats ont été obtenus à la fois en milieu complexe et en milieu défini, que ce soit à l'échelle du laboratoire ou à l'échelle d'un fermenteur de 800 litres; les cinétiques de croissance et de production d'ADN plasmidique sont de plus tout à fait comparables (cf. figures 6 et 7).

**TABLEAU 4**

| | **Milieu complexe** | | **Milieu défini** |
|---|---|---|---|
| | **Fermenteur de 2 ou 71** | **Fermenteur de 8001** | **Fermenteur de 21** |
| **durée de fermentation (heure)** | 40 | 39 | 48 |
| **µ h-1** | 0.130 | 0.132 | 0.124 |
| **DO (600nm)** | 114 | 100 | 94 |
| **X g/l** | 44 | 37 | 30 |
| **ADN plasmidique (mg/l milieu)** | 115 | 100 | 100 |
| **ADN plasmidique (mg/gX)** | 2.6 | 2.7 | 3.3 |
| X=biomasse (poids de cellules sèches) | | | |

Les résultats globaux font ressortir que :
- le changement d'échelle du fermenteur de 2 litres à celui de 800 litres peut être effectué sans problème aucun,
- l'oxygène consommé est fortement corrélé à la biomasse produite (1.08 g 02/g biomasse produite),
- le plasmide est stable pendant au moins 50 générations sans pression de sélection,
- on peut obtenir une biomasse élevée, supérieure à 40g de cellules sèches/litre, en milieu complexe,
- la production d'ADN plasmidique atteint les 100 mg d'ADN surenroulé / 1 de milieu,
- il existe une très bonne corrélation entre la production d'ADN et la biomasse: on peut estimer la production à (1 mg d'ADN plasmidique / unité de DO, ou bien (2.7 mg d'ADN plasmidique / g de biomasse, et ceci quelle que soit la durée de fermentation,
- l'utilisation d'un milieu défini permet aussi d'atteindre une biomasse (30 g de cellules sèches/1) et une production d'ADN plasmidique (100 mg/l) élevées, et ceci sans aucune perte de productivité.

### EXEMPLE 6:

### Transfert de pXL2774 dans les cellules animales, in vitro et in vivo.

### 6.1. Transfert in vitro de pXL2774 dans les cellules animales

La capacité du plasmide minimal pXL2774 à transfecter différentes lignées cellulaires a été testée in vitro, tant sur des cellules d'origine humaine que d'origine murine. Le plasmide pXL2784 a été utilisé comme témoin. Il contient la même cassette d'expression eucaryote (promoteur CMV-luciférase-polyA) que pXL2774 mais c'est un dérivé de ColE1 de 6,4kb qui comprend le gène conférant la résistance à la kanamycine chez E. coli.

Les cellules testées sont les suivantes:

| **Cellules** | **Type** | **Réf Atcc/réf. Biblio** |
|---|---|---|
| 3LL | Carcinome pulmonaire de souris | |
| NIH 3T3 | Fibroblastes d'embryon de souris | CCL92 |
| 293 | Cellules rénales d'embryon humain transformees par l'adenovirus type 5 | CRL1573 |
| HeLa | Carcinome humain du col de l'utérus | CCL2 |
| Caco-2 | Adenocarcinome humain du colon | HTB37 |
| H460 | Carcinome humain du poumon non a petites cellules | HTB177 |
| ECV 304 | Cellules endotheliales humaines de cordon ombilical | Takahashi et al.,1990 |

Les conditions de transfection ont été les suivantes :
J-1: Ensemencement des cellules à la densité de 100 000 cellules par puits de 2 cm2 (plaque 24 puits) en milieu DMEM (Dulbecco's modified Eagle Médium) supplémenté par 10 % de sérum de veau foetal (SVF).
J-3: Transfection des cellules, par 10 µl d'une solution de transfection contenant: 0,5 µg d'ADN, 150mM NaCl, 5 % glucose et 3 nmoles de lipofectant RPR120 535 par µg d'ADN, dans 250 µl de milieu de culture, additionné ou non de 10 % de SVF. Après une incubation de 2 heures, le milieu est remplacé par 500 µl de milieu DMEM additionné de 10 % de SVF.
J-4: Renouvellement du milieu de culture
J-5: Lavage des cellules au PBS puis lyse par 100 µl de tampon de lyse Promega (Promega Cell Lysis Buffer E153 A). Le dosage de l'activité luciférase s'effectue dans un lummomètre Lumat LB 9501 (Berthold) sur 10 µl de lysat, avec une durée d'intégration de 10 secondes. Le réactif utilisé est celui de Promega (Promega Luciferase Assay Substrate). Les résultats, rassemblés dans les tableaux suivants, sont exprimés en RLU (Relative Lights Unit) pour 10 µl de lysat (moyenne de mesure sur 4 puits). Les coefficients de variation (CV) sont aussi indiqués.

Les résultats de transfections en absence de sérum sont présentés ci-dessous.

| | **TYPES CELLULAIRES** | | | |
|---|---|---|---|---|
| | NIH 3T3 | 3LL | 293 | |
| **pXL 2774** | 37 763 380 16 | 559 270 25 | 1 884 200 73 | RLU CV |
| **pXL2784** | | 113 764 24 | 1 723 546 101 | RLU CV |

| TYPES CELLULAIRES | | | | |
|---|---|---|---|---|
| | | | | |
| | HeLa | CaCo2 | H460 | ECV304 |
| pXL 2774 | 11000 000 15 | 1108422 14 | 1 459 501 5 | 36450 23 |
| pXL2784 | 557930 87 | 93 610 40 | 7563 11 | 168795 40 |

Les résultats de transfections en présence de sérum (10 %) sont présentés ci-dessous :

| TYPES CELLULAIRES | | | |
|---|---|---|---|
| | NIH 3T3 | 3LL | 293 |
| pXL 2774 | 50 612 590 12 | 566 377 18 | 992 500 59 |
| PXL2784 | 12 693 780 38 | 436 704 12 | 2 300 000 47 |

| TYPES CELLULAIRES | | | |
|---|---|---|---|
| | HeLa | H460 | ECV304 |
| pXL 2774 | 9490000 25 | 857385 16 | 18021 30 |
| PXL2784 | 1 508 480 23 | 433023 27 | 32074 z47 |

Ces résultats font ressortir la capacité de pXL2774 à transfecter, in vitro, de façon efficace différents types cellulaires d'origine aussi bien murine qu'humaine. L'expression du gène rapporteur luc permet de montrer que son efficacité de transfection est au moins aussi bonne que celle d'un plasmide "classique", dérivé de ColE1. qui porte la même cassette d'expression de la luciférase.

### 6.2. Transfert in vivo, chez l'animal (souris), de pXL2774

### a) Modèle 1: ADN nu dans le muscle tibial crânial de souris

L'ADN plasmidique nu, en solution dans "glucose 5 %, NaCl 150mM", est injecté dans le muscle tibial crânial de souris OF1. Les muscles sont prélevés 7 jours après injection, hachés, homogénéisés dans 750 µl de tampon de lyse (Cell Lysis Buffer Promega E153A) puis centrifugés à 20 000 ¥ g pendant 10 minutes.

Le dosage d'activité luciférase est réalisé sur 10 µl surnageant après addition de 50 µl de réactif (Promega Luciferase Assay Substrate). La lecture s'effectue sur le luminomètre Lumat LB9501 (Berthold) avec une durée d'intégration de 10 secondes.

Les résultats sont présentés dans le Tableau ci-dessous.

| Plasmide | pXL2784 | pXL 2774 | pXL2784 | pXL 2774 |
|---|---|---|---|---|
| nombre de muscles: | 8 | 8 | 10 | 10 |
| volume injecté (µl) : | 30 | 30 | 33 | 33 |
| µg d'ADN/muscle | 19 | 13,5 | 10 | 6,25 |

| RLU (pour10 µl) | | | | |
|---|---|---|---|---|
| Moyenne | 80 922 | 471733 | 35329 | 30569 |
| Ecart-type | 104 573 | 402602 | 37041 | 35774 |

Ces résultats indiquent qu'un plasmide à réplication conditionnelle comme pXL2774 est bien capable de transfecter des cellules musculaires de souris in vivo et ceci avec une efficacité comparable, voire supérieure à celle d'un plasmide "classique", dérivé de Co1E1 , qui porte la même cassette d'expression du gène de la luciférase.

### b) Modèle 2: modèle tumoral 3T3 HER2

Le modèle est le suivant:
- Souris de type Swiss/nude femelles adulte
- Tumeurs expérimentales induites après injection de 107 cellules 3T3 HER2 par voie sous-cutanée au niveau du flanc.
- L'injection du mélange de transfection est réalisée 7 jours après l'injection des cellules
   Solutions injectées: L'ADN est d'abord solubilisé dans le tampon. Après addition de tous les produits, le mélange contient, outre l'ADN, du NaCl (150mM) et du D-Glucose 5 % dans l'eau ou le tampon HEPES 5mM.
- Deux jours après l'injection, le tissu tumoral est prélevé, pesé puis haché et homogénéisé dans 750 µl tampon de lyse (Promega Cell Lysis Buffer E153 A). Après centrifugation (20 000 g pendant 10 minutes), on prélève 10 µl de surnageant qui permettent l'évaluation de l'activité luciférase. Cette activité est déterminée par mesure de l'émission lumineuse totale obtenue après mélange avec 50 µl de réactif (Promega Luciferase Assay Substrate) dans un luminomètre Lumat LB 9501 (Berthold) avec une durée d'intégration de 10 secondes.

L'activité résultante est exprimée en RLU (Relative Lights Units) estimée dans la totalité du surnageant de lyse tumorale.
Résultats :

| Tampon | Plasmide | | | Résultat RLU /tumeur | | +/n |
|---|---|---|---|---|---|---|
| H2O ou HEPES | référence | µg/ tumeur | [ADN] finale ds sol.inj. | moyenne | écart-type | |
| HEPES | pXL2784 | 10 | 0,5 µg/µl | 744150 | 682 434 | 6/6 |
| | pXL2774 | 10 | 0,5 µg/µl | 1 016 380 | 1 322 500 | 5/6 |
| H2O | pXL2784 | 24 | 0,6 µg/µl | 2 906 073 | 1 745 857 | 8/8 |
| | pXL2774 | 16,8 | 0,4 µg/µl | 4 292 043 | 4 995 187 | 6/6 |
| H2O | pXL2784 | 7,5 | 0,3 µg/µl | 702 554 | 552207 | 6/7 |
| | pXL2774 | 5 | 0,2 µg/µl | 3 413 430 | 4 000 875 | 6/6 |

Ces résultats indiquent qu'un plasmide à réplication conditionnelle, comme pXL2774, est bien capable de transfecter des cellules tumorales de souris in vivo et ceci avec une efficacité au moins comparable à celle d'un plasmide "classique", dérivé de ColE1, qui porte la même cassette d'expression du gène de la luciférase.

Ces différentes expériences ont permis de démontrer que les plasmides à réplication conditionnelle, et plus particulièrement pXL2774, présentaient bien les caractéristiques de transfection de cellules animales indispensables à une utilisation en thérapie génique. Plus précisément, il a été montré :
1) la capacité de pXL2774 à transfecter efficacement, in vitro, différents types cellulaires, d'origine humaine ou murine;
2) la capacité de pXL2774 à transfecter, in vivo, le muscle de la souris;
3) la capacité de pXL2774 à transfecter, in vivo, des cellules tumorales implantées chez la souris

Les expériences d'électrotransformation, de fermentation et de transfection ont donc permis de valider les plasmides à réplication conditionnelle comme vecteur utilisables en thérapie génique en montrant
i) qu'il ne se répliquaient pas de façon détectable dans une souche d'E.coli n'exprimant pas le gène pir (origine de réplication conditionnelle)
ii) qu'ils pouvaient être produits à une échelle compatible avec une production industrielle, dans un milieu qui peut être totalement défini et qui ne contient pas d'antibiotiques;
iii) que ces plasmides pouvaient transfecter, in vitro et surtout in vivo des cellules de mammifères.

### EXEMPLE 7:

### Production de Protéines recombinantes in vitro

### 7.1. Construction du vecteur d'expression

Afin de montrer la faisabilité d'une telle approche, nous avons construit un vecteur d'expression selon les critères décrits précédemment (exemples 2 et 3). Ce vecteur, pXL3056, contient:
1) la partie bactérienne qui comprend l'origine de réplication conditionnelle (ori gamma), le fragment cer de ColE1 et le gène assurant la sélection chez la bactérie (sup)
2) la cassette d'expression, basée sur le système décrit par Studier (Studier et al., 1990), comprend le promoteur du gène 10 du bactériophage T7, l'opérateur lacO, le gène codant pour le aFGF 154 (acidic Fibroblast Growth factor ou facteur de croissance acide des fibroblastes, forme comportant 154 acides aminés) (Jaye et al., 1986), le terminateur TF du bactériophage T7. Cette cassette d'expression est identique à celle présente sur le plasmide pXL2434 décrit dans la demande WO96/08572.

La construction de pXL3056 est présentée sur la figure 8. Le fragment EcoRI-BgIII de pXL2434 (1,1kb) contenant la cassette d'expression du aFGF est cloné dans le vecteur à réplication conditionnelle pXL2979 (fragment purifié de 1,1 kb) aux sites BgIII et EcoRI pour générer pXL3056.

pXL2979 résulte de la ligature de 3 fragments: i)fragment AccI-XbaI de pXL2730 (0,8kb, qui apporte ori gamma et cer) ü) fragment NarI-SalI de pXL2755 (0,18 kb, qui apporte le gène sup Phe) iii) fragment SalI-SpeI de pXL2660 (1,5 kb qui apporte le gène conférant la résistance à la kanamycine).

pXL2660 résulte du clonage du fragment PstI de 1,2kb de pUC4K (Vieira et Messing, 1982) dans pMTL22 (Chambers et al., 1988) linéarisé par PstI.

### 7.2. Obtention de la souche d'expression

Le plasmide pXL3056 est introduit par transformation dans la souche XAC-1pir-116. La souche résultante est ensuite transformée par le plasmide PT7po123 (Mertens et al., 1995), à 30°C. Afin d'exprimer le gène d'intérêt sous contrôle du promoteur T7, la bactérie doit contenir, dans son génome, sur un plasmide ou un bactériophage, une cassette permettant l'expression de l'ARN polymérase du bactériophage T7. Dans l'exemple décrit, nous avons utilisé le plasmide PT7po123, compatible avec les dérivés de R6K tel pXL3056, et qui permet l'expression inductible par la température de l'ARN polymérase bactériophage T7. On peut toutefois envisager aussi de lysogéniser la souche XAC-1pir-116 par lambda DE3 (Studier et al., 1990) afin de ne conserver qu'un plasmide et induire la production de l'ARN polymérase de T7 plutôt par l'IPTG que par la température.

### 7.3. Expression du aFGF

La souche XAC-1pir-116 (pXL3056+PT7po123) est cultivée à 30°C, en milieu minimum M9 additionné de 0,2 % de casaminoacides (DIFCO) et de kanarnycine (25µg/ml), jusqu'à une densité optique à 600 nm de 0,6-1. La moitié de la culture est ensuite placée à 42°C (induction de l'ARN polymérase de T7) alors que l'autre moitié reste à 30°C (témoin négatif). La même expérience est menée avec la souche XAC-1pir-116(pXL3056+pUC4K) qui constitue un témoin d'expression du aFGF en absence d'ARN polymérase de T7.

Les résultats obtenus sont présentés sur la figure 9. Ils montrent que la production de aFGF est comparable ou supérieure à celle observée avec BL21(DE3)(pXL2434) (WO96/08572) ce qui montre bien les potentialités des plasmides à réplication conditionnelle pour l'expression de protéines recombinantes in vitro, notamment chez E. Coli.

### EXEMPLE 8:

### Construction d'un vecteur pCOR exprimant une protéine p53 sauvage ou hybride.

Cet exemple décrit la construction de vecteurs à réplication conditionnelle selon l'invention contenant un acide nucléique codant pour une protéine p53. Ces vecteurs sont utilisables pour restaurer une activité de type p53 dans des cellules déficientes (mutées, délétées), telles que notamment les cellules tumorales.

La cassette d'expression eucaryote contient les éléments suivants :
1) promoteur précoce CMV "immediate early" (positions -522 à +72) suivi de la séquence leader du gène de la thymidine kinase du virus herpes simplex type I (position -60 à +1 du gène, en faisant référence à la séquence de l'article McKnight, S.tL. (1980) Nucleic Acids Res. 8:5949-5964) ;
2) un acide nucléique codant pour la protéine p53 sauvage ou pour un variant de p53 tel que décrit dans la demande PCT/FR96/01111 (variant V325K= V325 avec une séquence Kozak à l'ATG) ;
3) la séquence de polyadénylation polyA de SV40.

Ces éléments ont été placés sous forme d'un fragment AscI- XbaI sur le vecteur pCOR pXL2988 entre les sites BssHII et SpeI. pXI2988 est identique à pXL2979 (exemple 7.1.) mis à part la présence d'un élément supplémentaire, une séquence capable de former une triple hélice d'ADN composée de 17 fois le trinucléotide GAA, placée à coté de l'origine de réplication gamma.

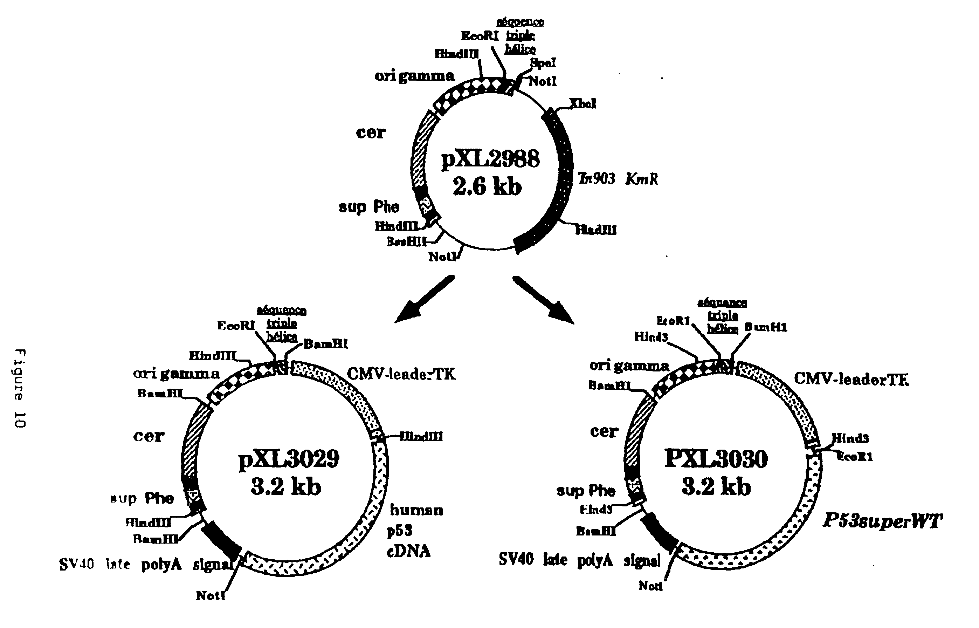

Les plasmides résultant sont nommés pXL3029 et 3030 (Figure 10).

La fonctionnalité de ces constructions a été vérifiée in vitro sur cellules p53-SAOS2 en culture par mesure de l'activité d'activateur transcriptionnel de p53 ou p53superWT.

### BIBLIOGRAPHIE

Alting-Mees, M. A., J. A. Sorge, and J. M. Short. 1992. Methods Enzymol. 216:483-495.
Blum, P., D. Holzschu, H. S. Kwan, D. Riggs, and S. Artz. 1989. J. Bacteriol. 171:538-546.
Brosius, J. 1989. Methods Enzymol. 216:469-483.
Chambers, S. P., S. E. Prior, D. A. Barstow, and N. P. Minton. 1988. Gene 68:139-149.
Chung, C. T., and R. H. Miller. 1988. Nucleic Acids Res. 16:3580.
Colloms, S. D., P. Sykora, G. Szatmari, and D. J. Sherrat. 1990 J. Bacteriol. 172:6973-6980.
Datta, N., and P. Kontomichalou. 1965. Nature 208:239-241.
Dickely, F., D. Nilsson, E. B. Hansen, and E. Johansen. 1995. Mol. Microbiol. 15:839-847.
Filutowicz, M. , S. Dellis, I. Levchenko, M. Urh, F. Wu, and D. York. 1994. Prog. in Nucleic Acid Res. and Mol. Biol. 48:239-273.
Gibson, T. J. 1984. Ph.D Thesis. University of Cambridge.
Greener, A., M. Filutowicz, M. McEachem, and D. Helinski. 1990. Mol. Gen. Genet. 224:24-32.
Herrero, M., V. de Lorenzo, and K. N. Timmis. 1990. J. Bacteriol. 172: 6557-6567.
Hodgson, C. P. 1995. Bio/Technology 13:222-225.
Inuzuka, M., and Y. Wada. 1985. EMBO J. 4:2301-2307.
Jaye, M. et al., (1986) Science 233: 541-5.
Kleina, L. G., J. M. Masson, J. Normanly, J. Abelson, and and J. H. Miller. 1990. J. Mol. Biol. 213:705-717.
Kowalczykowski, S. C., and A. K. Eggleston. 1994. Annu. Rev. Biochem. 63:9991-10043.
Leung, D. W., E. Chen, G. Cachianes, and D. V. Goeddel. 1985. DNA 4:351-355.
Maniatis, T., E. F. Fritsch, and J. Sambrook. 1989. Cold Spring Harbor Laboratory, Cold Spring Harbor, N.Y.
Meinnel, T., E. Schmitt, Y. Mechulam, and S. Blanquet. 1992. J. Bacteriol. 174:2323-2331.
Mertens, N., E. Remaut and W. Fiers. (1995) Bio/Technology 13:175-179.
Messing, J., and J. Vieira. 1982. Gene 19: 269-276.
Metcalf, W. W., W. Jiang, and B. L. Wanner. 1994. Gene 138:1-7.
Miller, V. L., and J. J. Mekalanos. 1988. J. Bacteriol. 170:2575-2583.
Normanly, J., J. M. Masson, L. G. Kleina, J. Abelson, and J. H. Miller. 1986. Proc. Natl. Acad. Sci. USA 83:6548-6552.
Normanly, J., L. G. Kleina, J. M. Masson, J. Abelson, and J. H. Miller. 1990. J. Mol. Biol. 213: 719-726.
Roca, J. 1995. TIBS 20:156-160.
Saïki, R. K., S. Scharf, F. Faloona, K. B. Mullis, G. T. Horn, H. A. Erlich, and N. Arnheim. 1985. Science 230:1350-1354.
Sanger, F., S. Nicklen, and A. R. Coulson. 1977. Proc. Natl. Acad. Sci. USA 74:5463-5467.
Sawadogo, M., and M. W. Van Dyke. 1991. Nucleic Acids Res. 19: 674.
Scott, J. R. 1984. Microbiol. Rev. 48:1-23.
Simoes, D. A., M. Dal Jensen, E. Dreveton, M. O. Loret, S. Blanchin-Roland, J. L. Uribelarrea, and J. M. Masson. 1991. Ann. N. Y. Acad. Sci. 646:254-258.
Simon, R., U. Priefer, and A. Pühler. 1983. Bio/Technology 1:784-791.
Sinha, N. D., J. Biernat, J. McManus, and H. Kôster. 1984. Nucleic Acids Res. 12:4539-4557.
Stirling, C. J., G. Stewart, and D. J. Sherrat. 1988. Mol. Gen. Genet. 214:80-84.
Stirling, C. J., S. D. Colloms, J. F. Collins, G. Szatmari, and D. J. Sherrat. 1989. EMBO J. 8:1623-1627.
Studier, F. W., A. H Rosenberg., J. J Dunn, and J. W. Dubendorff (1990). Methods Enzymol 185: 60-89.
Summers, D. K., and D. J. Sherrat. 1984. Cell 36:1097-1103.
Takahashi, K., Y. Sawasaki, J. Hata, K. Mukai and T. Goto.(1990) In Vitro Cell Dev. Biol. 26: 265-74
Vieira, J., and J. Messing. 1982. Gene 19:259-268.
Wiechelman, K., R. Braun, and J. Fitzpatrick. 1988. Anal. Biochem. 175:231-237.
Yanisch-Perron, C. Vieira. and J. Messing (1985) Gene 33: 103-119 13

## Revendications

1. Molécule d'ADN de forme circulaire, utile en thérapie génique, comprenant au moins une séquence nucléique d'intérêt, et également les signaux de régulation de son expression, dont la transcription et éventuellement la traduction dans la cellule cible eucaryote génèrent des produits ayant un intérêt thérapeutique, vaccinal, agronomique ou vétérinaire, **caractérisée en ce que** :
a. la région permettant sa réplication comprend une origine de réplication conditionnelle issue d'un plasmide ou d'un bactériophage dont la fonctionnalité dans une cellule procaryote hôte requiert la présence d'au moins une protéine initiatrice de réplication spécifique dudit plasmide ou bactériophage et étrangère à ladite cellule procaryote hôte,
b. ladite molécule d'ADN ne comporte pas ladite protéine initiatrice de réplication,
c. ladite molécule d'ADN comporte un marqueur de sélection de taille réduite différent d'un gène conférant la résistance à un antibiotique, et
d. ladite molécule d'ADN ne se réplique que dans les cellules pouvant complémenter ladite protéine initiatrice.

2. Molécule d'ADN selon la revendication 1 **caractérisée en ce que** ladite origine de réplication conditionnelle est issue d'un plasmide ou bactériophage qui possède une origine de réplication, représentée par plusieurs itérons, et qui code pour au moins une protéine spécifique, conditionnant la fonctionnalité de son origine de réplication.

3. Molécule d'ADN selon la revendication 1 ou 2 **caractérisée en ce que** l'origine de réplication conditionnelle peut être issue des plasmides ou bactériophages suivants RK2, R6K, R1, pSC101, Rtsl, F, RSF1010, P1, P4, lambda, Phi82 et Phi80.

4. Molécule d'ADN selon l'une des revendications 1 à 3 **caractérisée en ce que** ladite origine de réplication est issue du plasmide bactérien R6K.

5. Molécule d'ADN selon l'une des revendications précédentes **caractérisée en ce que** ladite origine de réplication est constituée en tout ou partie par l'origine de réplication γ du plasmide R6K.

6. Molécule d'ADN selon l'une des revendications précédentes **caractérisée en ce que** ladite origine de réplication est représentée en tout ou partie par la séquence SEQ ID N°1.

7. Molécule d'ADN selon l'une des revendications précédentes, **caractérisée en ce que** la région permettant la sélection des cellules hôtes incorporant ladite molécule d'ADN est représentée en tout ou partie par une cassette d'expression d'un ARN de transfert suppresseur de codons spécifiques.

8. Molécule d'ADN selon la revendication 7, **caractérisée en ce qu'**il s'agit d'une cassette d'expression d'un ARNt suppresseur de codons ambre (TAG).

9. Molécule d'ADN selon l'une des revendications précédentes, **caractérisée en ce qu'**elle comprend en outre une région cible de recombinase site-spécifique.

10. Molécule d'ADN selon la revendication 9, **caractérisée en ce que** cette région cible de recombinase site-spécifique est choisie parmi les résolvases des transposons, Tn3, Tn21 ou Tn522 ; l'invertase du bactériophage mu, les resolvases de plasmides, comme celle codée par le fragment par de RP4, les recombinases de la famille de l'intégrase du bactériophage lambda, comme les intégrases de lambda, phi80, P22, HP1, l'intégrase Cre de P1, l'intégrase du plasmide pSAM2 , la FLP recombinase du plasmide 2µ, et les recombinases XerC et XerD d'E. Coli.

11. Molécule d'ADN selon la revendication 9 ou 10, **caractérisée en ce que** cette région cible de recombinase site-spécifique comprend tout ou partie du fragment *cer* de ColEl ou d'un fragment plus petit et possédant les mêmes propriétés.

12. Molécule d'ADN selon l'une des revendications précédentes, **caractérisée en ce que** la séquence d'acide nucléique d'intérêt code pour une protéine d'intérêt pharmaceutique, agroalimentaire ou utilisable en biocatalyse.

13. Molécule d'ADN selon la revendication 12 **caractérisée en ce que** la séquence d'acide nucléique d'intérêt code pour une protéine choisie parmi les enzymes, les dérivés sanguins, les hormones, les lymphokines : interleukines, interférons, TNF, les facteurs de croissance, les neurotransmetteurs ou leurs précurseurs ou enzymes de synthèse, les facteurs trophiques comme BDNF, CNTF, NGF, IGF, GMF, aFGF, bFGF, NT3, NT5, les apolipoprotéines comme ApoAI, ApoAIV, ApoE, la dystrophine ou une minidystrophine, les gènes suppresseurs de tumeurs comme p53, Rb, RaplA, DCC, k rev, les gènes codant pour des facteurs impliqués dans la coagulation comme les Facteurs VII, VIII, IX, les gènes suicides comme la thymidine kinase, la cytosine désaminase, tout ou partie d'une immunoglobuline naturelle ou artificielle comme le Fab, le ScFv, un A RN ligand, une séquence anti sens ou des peptides antigéniques spécifiques du virus d'Epstein Barr, du virus HIV, du virus de l'hépatite B, du virus de la pseudo rage, ou encore spécifiques de tumeurs.

14. Procédé de production de molécule d'ADN de forme circulaire selon l'une des revendications 1 à 13, **caractérisé en ce que** l'on cultive une cellule hôte contenant au moins:
a. une molécule d'ADN selon l'une des revendications 1 à 13 comprenant une origine de réplication dont la fonctionnalité dans la cellule hôte requiert la présence d'au moins une protéine initiatrice de réplication spécifique et étrangère à ladite cellule hôte, et
b. ladite protéine, exprimée *in situ* ou non, conditionnant la fonctionnalité de ladite origine de réplication, spécifique et étrangère à ladite cellule hôte,
dans des conditions permettant la sélection des cellules hôtes transformées par lesdites molécules d'ADN.

15. Procédé selon la revendication 14 **caractérisé en ce que** le gène codant pour la protéine initiatrice de la réplication est présent sur un réplicon annexe ou incorporé dans le génome de ladite cellule.

16. Procédé selon la revendication 14 ou 15 **caractérisé en ce qu'**il s'agit d'une molécule d'ADN incorporant une origine de réplication telle que définie dans les revendications 4 à 6 et **en ce que** ladite protéine est la protéine Π du plasmide R6K ou diffère de cette protéine par modification de sa séquence codante ou constitue un fragment de cette protéine tout en ayant la même activité.

17. Procédé selon la revendication 16 **caractérisé en ce que** la protéine Π ou l'un de ses dérivés est exprimée in situ à partir du gène pir représenté en SEQ ID N°2 ou toute séquence différent de SEQ ID N°2 en raison d'une dégénérescence du code génétique, ou toute séquence hybridant avec ces séquences ou des fragments de celles ci présents dans la cellule hôte et dont le produit possède l'activité de la protéine spécifique initiatrice de la réplication.

18. Procédé selon la revendication 17 **caractérisé en ce que** la protéine est exprimée à partir du gène pir 116 incorporé dans le génome de ladite cellule.

19. Procédé selon l'une des revendications 14 à 18 **caractérisé en ce que** l'un des gènes indispensables dans les conditions de culture choisies, de la cellule hôte, contient un codon spécifique, reconnaissable par l'ARNt suppresseur sélectionné au niveau de la molécule d'ADN.

20. Procédé selon la revendication 19 **caractérisé en ce que** ce gène contient un codon ambre TAG.

21. Procédé selon l'une des revendications 14 à 20 **caractérisé en ce que** la cellule est choisie parmi les souches d'E. coli.

22. Procédé selon l'une des revendications 14 à 21 **caractérisé en ce que** la cellule est issue de la souche E. coli XAC 1.

23. Procédé selon l'une des revendications 15 à 22 **caractérisé en ce qu'**il met en oeuvre la souche E. coli XAC 1, intégrant dans son génome le gène pir 116.

24. Cellule recombinante transformée par au moins une molécule d'ADN selon l'une des revendications 1 à 13.

25. Composition pharmaceutique comprenant une ou plusieurs molécule(s) d'ADN selon l'une des revendications 1 à 13.

26. Molécule d'ADN selon l'une des revendications 1 à 13 à titre de médicament.

27. Utilisation d'une molécule d'ADN selon l'une des revendications 1 à 13 pour la production de protéines recombinantes in vitro.

28. Molécule selon la revendication 1 **caractérisée en ce que** la séquence nucléique d'intérêt code pour une protéine p53 sauvage ou modifiée.

29. Molécule selon la revendication 1 **caractérisée en ce que** la séquence nucléique d'intérêt code pour une thymidine kinase.

30. Procédé selon la revendication 21 **caractérisé en ce que** la cellule est une souche de E. coli endA 1.

31. Molécule d'ADN selon l'une des revendications 1 à 13 **caractérisée en ce qu'**elle comprend en outre une séquence capable d'interagir spécifiquement avec un ligand.

32. Molécule d'ADN selon la revendication 13 **caractérisée en ce que** l'acide nucléique d'intérêt code pour le facteur de croissance acide des fibroblastes (FGFa).

33. Molécule d'ADN selon la revendication 32 à titre de médicament.

34. Utilisation de molécule d'ADN selon l'une des revendications 1 à 13 pour la fabrication d'un médicament utile dans le traitement du cancer.

## Claims

1. Circular DNA molecule, which is useful in gene therapy, this molecule comprising at least one nucleic acid sequence of interest, and also the signals for regulating its expression, whose transcription, and optionally the translation in the eukaryotic target cell, generate products of therapeutic, vaccinal, agronomic or veterinary value, **characterized in that**:
a. the region which allows its replication comprises a conditional origin of replication which is derived from a plasmid or a bacteriophage and whose functionality in a prokaryotic host cell requires the presence of at least one replication-initiating protein which is specific to the said plasmid or bacteriophage and which is foreign to the said prokaryotic host cell,
b. the said DNA molecule does not contain the said replication-initiating protein,
c. the said DNA molecule contains a selection marker of reduced size other then a gene conferring resistance to an antibiotic, and
d. the said DNA molecule replicates only in cells able to complement the said initiating protein.

2. DNA molecule according to Claim 1,
**characterized in that** the said conditional origin of replication is derived from a plasmid or bacteriophage which possesses an origin of replication, represented by several iterons, and which codes for at least one specific protein, which conditions the functionality of its origin of replication.

3. DNA molecule according to Claim 1 or 2, **characterized in that** the conditional origin of replication may be derived from the following plasmids or bacteriophages; RK2, R6K, R1, pSC101, Rts1, F, RSF1010, P1, P4, lambda, Phi82 and Phi80.

4. DNA molecule according to one of Claims 1 to 3, **characterized in that** the said origin of replication is derived from the bacterial plasmid R6K.

5. DNA molecule according to one of the preceding claims, **characterized in that** the said origin of replication consists entirely or partially of the y origin of replication of the plasmid R6K.

6. DNA molecule according to one of the preceding claims, **characterized in that** the said origin of replication is represented entirely or partially by the sequence SEQ ID No. 1.

7. DNA molecule according to one of the preceding claims, **characterized in that** the region allowing the selection of host cells which incorporate the said DNA molecule is represented entirely or partially by an expression cassette of a suppressor transfer RNA for specific codons.

8. DNA molecule according to Claim 7,
**characterized in that** it is an expression cassette of a suppressor tRNA for amber codons (TAG).

9. DNA molecule according to one of the preceding claims, **characterized in that** it also comprises a target region for site-specific recombinase.

10. DNA molecule according to Claim 9, **characterized in that** this target region for site-specific recombinase is chosen from the resolvases of transposons Tn3, Tn21 or Tn522; the invertase of the bacteriophage mu, the plasmid resolvases, such as that encoded by the RP4 par fragment, the recombinases of the bacteriophage lambda integrase family, such as the integrases from lambda, phi80, P22 and HP1, the P1 Cre integrase, the integrase from the plasmid pSAM2, the FLP recombinase from the 2µ plasmid, and the E. coli recombinases XerC and XerD.

11. DNA molecule according to Claim 9 or 10, **characterized in that** this target region for site-specific recombinase comprises all or part of the cer fragment from ColE1 or of a smaller fragment with the same properties.

12. DNA molecule according to one of the preceding claims, **characterized in that** the nucleic acid sequence of interest codes for a protein of pharmaceutical or agrifood interest or a protein which can be used in biocatalysis.

13. DNA molecule according to Claim 12,
**characterized in that** the nucleic acid sequence of interest codes for a protein chosen from enzymes, blood derivatives, hormones, lymphokines: interleukins, interferons, TNF, growth factors, neurotransmitters or their precursors or synthetic enzymes, trophic factors such as BDNF, CNTF, NGF, IGF, GMF, aFGF, bFGF, NT3, NT5, apolipoproteins such as ApoAI, ApoAIV and ApoE, dystrophin or a minidystrophin, tumour-suppressing genes such as p53, Rb, RaplA, DCC, k-rev, genes coding for factors involved in coagulation such as factors VII, VIII and IX, suicide genes such as thymidine kinase and cytosine deaminase, all or part of a natural or artificial immunoglobulin such as Fab, ScFv, an RNA ligand, an antisense sequence or specific antigenic peptides of Epstein-Barr virus, HIV virus, hepatitis B virus or pseudorabies virus, or alternatively antigenic peptides which are tumour-specific.

14. Process for the production of a circular DNA molecule according to one of Claims 1 to 13, **characterized in that** a host cell containing at least:
a. one circular DNA molecule according to one of Claims 1 to 13 comprising an origin of replication whose functionality in the host cell requires the presence of at least one specific replication-initiating protein which is foreign to the said host cell, and
b. the said protein, which is or is not expressed in situ, conditions the functionality of the said origin of replication, is specific and is foreign to the said host cell, is cultured under conditions which allow the selection of the host cells transformed by the said DNA molecules.

15. Process according to Claim 14,
**characterized in that** the gene coding for the replication-initiating protein is present on a subsidiary replicon or a replicon incorporated into the genome of the said cell.

16. Process according to Claim 14 or 15, **characterized in that** the DNA molecule is a DNA molecule which incorporates an origin of replication as defined in claims 4 to 6 and **in that** the said protein is the n protein of the plasmid R6K or differs from this protein by modification of its coding sequence or constitutes a fragment of this protein while having the same activity.

17. Process according to Claim 16, **characterized in that** the n protein or one of its derivatives is expressed in situ from the pir gene represented in SEQ ID No. 2 or any sequence which differs from SEQ ID No. 2 on account of degeneracy of the genetic code, or any sequence which hybridizes with these sequences or fragments thereof present in the host cell and whose product has the activity of the specific replication-initiating protein.

18. Process according to Claim 17,
**characterized in that** the protein is expressed from the pir 116 gene incorporated into the genome of the said cell.

19. Process according to one of Claims 14 to 18, **characterized in that** one of the host-cell genes which are essential under the chosen culture conditions contains a specific codon, which is recognizable by the selected suppressor tRNA in the DNA molecule.

20. Process according to Claim 19,
**characterized in that** this gene contains an amber TAG codon.

21. Process according to one of Claims 14 to 20, **characterized in that** the cell is chosen from strains of E. coli.

22. Process according to one of Claims 14 to 21, **characterized in that** the cell is derived from the E. coli strain XAC 1.

23. Process according to one of Claims 15 to 22, **characterized in that** it uses the E. coli strain XAC 1, incorporating the gene pir 116 into its genome.

24. Recombinant cell transformed with at least one DNA molecule according to one of Claims 1 to 13.

25. Pharmaceutical composition comprising one or more DNA molecule(s) according to one of claims 1 to 13.

26. DNA molecule according to one of Claims 1 to 13 as a medicament.

27. Use of a DNA molecule according to one of Claims 1 to 13 for the in vitro production of recombinant proteins.

28. Molecule according to Claim 1,
**characterized in that** the nucleic acid sequence of interest codes for a wild-type or modified p53 protein.

29. Molecule according to Claim 1,
**characterized in that** the nucleic acid sequence of interest codes for a thymidine kinase.

30. Process according to Claim 21,
**characterized in that** the cell is an E. coli endA 1 strain.

31. DNA molecule according to one of Claims 1 to 13, **characterized in that** it also comprises a sequence capable of interacting specifically with a ligand.

32. DNA molecule according to Claim 13,
**characterized in that** the nucleic acid of interest codes for acidic fibroblast growth factor (aFGF).

33. DNA molecule according to Claim 32 as a medicament.

34. Use of a DNA molecule according to one of Claims 1 to 13, for manufacturing a medicinal product useful in the treatment of cancer.

## Patentansprüche

1. DNA-Molekül von zirkulärer Form, welches in der Gentherapie nützlich ist, umfassend wenigstens eine Nukleinsäuresequenz von Interesse und gleichfalls die Regulationssignale für deren Expression, deren Transkription und gegebenenfalls Translation in der eukaryotischen Zielzelle Produkte mit einem therapeutischen, in Zusammenhang mit Impfungen bestehenden, landwirtschaftlichen oder veterinärmedizinischen Nutzen erzeugen, **dadurch gekennzeichnet, dass**:
a. die Region, welche dessen Replikation erlaubt, einen konditionalen (abhängigen) Replikationsstartpunkt umfasst, welcher aus einem Plasmid oder einem Bakteriophagen stammt, dessen Funktionalität in einer prokaryotischen Wirtszelle die Anwesenheit von wenigstens einem Replikationsinitiationsprotein, welches für das Plasmid oder den Bakteriophagen spezifisch ist und in Bezug auf die prokaryotische Wirtszelle fremd ist, erfordert,
b. das DNA-Molekül das Replikationsinitiationsprotein nicht umfasst,
c. das DNA-Molekül einen Selektionsmarker von verringerter Größe, welcher von einem Gen, welches Resistenz gegen ein Antibiotikum verleiht, verschieden ist, umfasst und
d. das DNA-Molekül sich lediglich in Zellen, die das Initiationsprotein komplementieren können, repliziert.

2. DNA-Molekül nach Anspruch 1, **dadurch gekennzeichnet, dass** der konditionale Replikationsstartpunkt aus einem Plasmid oder Bakteriophagen stammt, welches bzw. welcher einen Replikationsstartpunkt aufweist, welcher durch mehrere Iterone (repetitive Sequenzen) repräsentiert wird, und welches bzw. welcher wenigstens ein spezifisches Protein kodiert, welches die Funktionalität von dessen Replikationsstartpunkt bedingt (konditioniert).

3. DNA-Molekül nach Anspruch 1 oder 2, **dadurch gekennzeichnet, dass** der konditionale Replikationsstartpunkt aus den folgenden Plasmiden oder Bakteriophagen stammen kann: RK2, R6K, R1, pSC101, Rtsl, F, RSF1010, P1, P4, lambda, Phi82 und Phi80.

4. DNA-Molekül nach einem der Ansprüche 1 bis 3, **dadurch gekennzeichnet, dass** der Replikationsstartpunkt aus dem bakteriellen Plasmid R6K stammt.

5. DNA-Molekül nach einem der vorangegangenen Ansprüche, **dadurch gekennzeichnet, dass** der Replikationsstartpunkt in seiner Gesamtheit oder zu einem Teil durch den Replikationsstartpunkt y des Plasmids R6K gebildet wird.

6. DNA-Molekül nach einem der vorangegangenen Ansprüche, **dadurch gekennzeichnet, dass** der Replikationsstartpunkt in seiner Gesamtheit oder zu einem Teil durch die Sequenz SEQ ID No. 1 angegeben wird.

7. DNA-Molekül nach einem der vorangegangenen Ansprüche, **dadurch gekennzeichnet, dass** die Region, welche die Selektion der Wirtszellen, welche das DNA-Molekül enthalten, ermöglicht, in ihrer Gesamtheit oder zu einem Teil durch eine Expressionskassette einer Suppressor-Transfer-RNA für spezielle Codons repräsentiert wird.

8. DNA-Molekül nach Anspruch 7, **dadurch gekennzeichnet, dass** es sich um eine Expressionskassette einer Suppressor-tRNA für Amber-Codons (TAG) handelt.

9. DNA-Molekül nach einem der vorangegangenen Ansprüche, **dadurch gekennzeichnet, dass** es außerdem eine Zielregion einer ortsspezifischen Rekombinase umfasst.

10. DNA-Molekül nach Anspruch 9, **dadurch gekennzeichnet, dass** diese Zielregion einer ortsspezifischen Rekombinase ausgewählt wird unter den Resolvasen der Transposons Tn3, Tn21 oder Tn522; der Invertase des Bakteriophagen mu, den Resolvasen von Plasmiden, wie jener, die durch das Fragment par von RP4 kodiert wird, den Rekombinasen aus der Familie der Integrase des Bakteriophagen lambda, wie den Integrasen von lambda, phi80, P22, HP1, der Integrase Cre von P1, der Integrase des Plasmids pSAM2, der FLP-Rekombinase des Plasmids 2µ und den Rekombinasen XerC und XerD von E. coli.

11. DNA-Molekül nach Anspruch 9 oder 10, **dadurch gekennzeichnet, dass** diese Zielregion einer ortsspezifischen Rekombinase die Gesamtheit oder einen Teil des cer-Fragments von ColE1 oder eines kleineres Fragments, welches die gleichen Eigenschaften aufweist, umfasst.

12. DNA-Molekül nach einem der vorangegangenen Ansprüche, **dadurch gekennzeichnet, dass** die Nukleinsäuresequenz von Interesse ein Protein von pharmazeutischem, agrarwirtschaftlichem Nutzen oder welches in der Biokatalyse einsetzbar ist, kodiert.

13. DNA-Molekül nach Anspruch 12, **dadurch gekennzeichnet, dass** die Nukleinsäuresequenz von Interesse ein Protein kodiert, welches ausgewählt wird unter den Enzymem, den aus Blut abgeleiteten Substanzen, den Hormonen, den Lymphokinen: Interleukinen, Interferonen, TNF, den Wachstumsfaktoren, den Neurotransmitttern oder deren Synthese-Vorstufen oder -Enzymen, den trophischen Faktoren, wie BDNF, CNTF, NGF, IGF, GMV, aFGF, bFGF, NT3, NT5, den Apolipoproteinen, wie ApoAI, ApoAIV, ApoE, Dystrophin oder einem Minidystrophin, den Tumorsuppressorgenen, wie p53, Rb, RaplA, DCC, k rev, den Genen, welche Faktoren kodieren, welche an der Gerinnung beteiligt sind, wie die Faktoren VII, VIII, IX, den Suizidgenen, wie Thymidinkinase, Cytosindesaminase, der Gesamtheit oder einem Teil eines natürlichen oder künstlichen Immunglobulins, wie Fab, ScFv, einem RNA-Ligand, einer Antisinn-Sequenz oder antigenen Peptiden, welche für das Epstein Barr-Virus, das HIV-Virus, das Hepatitis B-Virus, das Pseudotollwut-Virus spezifisch sind oder ferner für Tumore spezifisch sind.

14. Verfahren zur Herstellung eines DNA-Moleküls von zirkulärer Form nach einem der Ansprüche 1 bis 13, **dadurch gekennzeichnet, dass** man eine Wirtszelle, welche wenigstens enthält:
a. ein DNA-Molekül nach einem der Ansprüche 1 bis 13, welches einen Replikationsstartpunkt umfasst, dessen Funktionalität in der Wirtszelle die Anwesenheit von wenigstens einem spezifischen und in Hinblick auf die Wirtszelle fremden Replikationsinitiationsprotein erfordert, und
b. das spezifische und in Hinblick auf die genannte Wirtszelle fremde, *in situ* oder nicht *in situ* exprimierte Protein, welches die Funktionalität des Replikationsstartpunkts bedingt (konditioniert),
unter Bedingungen kultiviert, welche die Selektion der durch die DNA-Moleküle transformierten Wirtszellen erlauben.

15. Verfahren nach Anspruch 14, **dadurch gekennzeichnet, dass** das das Replikationsinitiationsprotein kodierende Gen auf einem Replikon vorhanden ist, welches an das Genom der Zelle angefügt oder in dieses inkorporiert ist.

16. Verfahren nach Anspruch 14 oder 15, **dadurch gekennzeichnet, dass** es sich um ein DNA-Molekül, welches einen Replikationsstartpunkt, wie in den Ansprüchen 4 bis 6 definiert, umfasst, handelt und dass das Protein das Protein n des Plasmids R6K ist oder sich von diesem Protein durch Modifizierung von dessen kodierender Sequenz unterscheidet oder ein Fragment dieses Proteins bildet, wobei dieses nach wie vor die gleiche Aktivität aufweist.

17. Verfahren nach Anspruch 16, **dadurch gekennzeichnet, dass** das Protein π oder das eine seiner Derivate in situ exprimiert wird ausgehend von dem in SEQ ID No. 2 angegebenen Gen pir oder einer jeglichen Sequenz, welche sich von SEQ ID No. 2 aufgrund einer Degeneration des genetischen Codes unterscheidet, oder einer jeglichen Sequenz, welche mit diesen Sequenzen hybridisiert, oder Fragmenten von jenen, die in der Wirtszelle vorhanden sind und deren Produkt die Aktivität des spezifischen Replikationsinitiationsproteins aufweist.

18. Verfahren nach Anspruch 17, **dadurch gekennzeichnet, dass** das Protein ausgehend von dem Gen pir 116, welches in das Genom der Zelle eingebaut ist, exprimiert wird.

19. Verfahren nach einem der Ansprüche 14 bis 18, **dadurch gekennzeichnet, dass** das eine der Gene, welche unter den gewählten Kulturbedingungen der Wirtszelle unentbehrlich sind, ein spezielles Codon enthält, welches durch die ausgewählte Suppressor-tRNA auf der Ebene des DNA-Moleküls erkannt werden kann.

20. Verfahren nach Anspruch 19, **dadurch gekennzeichnet, dass** dieses Gen ein Amber-Codon TAG enthält.

21. Verfahren nach einem der Ansprüche 14 bis 20, **dadurch gekennzeichnet, dass** die Zelle unter den Stämmen von E. coli ausgewählt wird.

22. Verfahren nach einem der Ansprüche 14 bis 21, **dadurch gekennzeichnet, dass** die Zelle aus dem E. coli-Stamm XAC 1 stammt.

23. Verfahren nach einem der Ansprüche 15 bis 22, **dadurch gekennzeichnet, dass** es den Stamm E. coli XAC 1, welcher in seinem Genom das Gen pir 116 enthält, einsetzt.

24. Rekombinante Zelle, welche durch wenigstens ein DNA-Molekül nach einem der Ansprüche 1 bis 13 transformiert ist.

25. Pharmazeutische Zusammensetzung, welche ein oder mehrere DNA-Molekül(e) nach einem der Ansprüche 1 bis 13 umfasst.

26. DNA-Molekül nach einem der Ansprüche 1 bis 13 als Arzneimittel.

27. Verwendung eines DNA-Moleküls nach einem der Ansprüche 1 bis 13 für die Herstellung von rekombinanten Proteinen in vitro.

28. Molekül nach Anspruch 1, **dadurch gekennzeichnet, dass** die Nukleinsäuresequenz von Interesse ein Wildtyp-p53-Protein oder ein modifiziertes p53-Protein kodiert.

29. Molekül nach Anspruch 1, **dadurch gekennzeichnet, dass** die Nukleinsäuresequenz von Interesse eine Thymidinkinase kodiert.

30. Verfahren nach Anspruch 21, **dadurch gekennzeichnet, dass** die Zelle ein E. coli-Stamm endA 1 ist.

31. DNA-Molekül nach einem der Ansprüche 1 bis 13, **dadurch gekennzeichnet, dass** es außerdem eine Sequenz, welche zur spezifischen Wechselwirkung mit einem Liganden in der Lage ist, umfasst.

32. DNA-Molekül nach Anspruch 13, **dadurch gekennzeichnet, dass** die Nukleinsäure von Interesse den sauren Wachstumsfaktor der Fibroblasten (FGFa) kodiert.

33. DNA-Molekül nach Anspruch 32 als Arzneimittel.

34. Verwendung eines DNA-Moleküls nach einem der Ansprüche 1 bis 13 für die Herstellung eines Arzneimittels, welches bei der Behandlung von Krebs nützlich ist.
